# EUROPEAN PATENT SPECIFICATION

(11) **EP 1 534 751 B1**
(45) Date of publication and mention of the grant of the patent: **14.04.2010**
(21) Application number: 03790928.0
(22) Date of filing: 27.08.2003
(51) Int. Cl.: C07K 14/78, A61K 38/39

(54) **OCULAR GENE THERAPY**
GENTHERAPIE DES AUGES
THERAPIE GENIQUE OCULAIRE

(30) Priority: 28.08.2002 US 406470 P
(43) Date of publication of application: 01.06.2005
(73) Proprietor: Novartis AG, 4056 Basel (CH); Novartis Pharma GmbH, 1230 Wien (AT)
(72) Inventor: CAMPOCHIARO, Peter, Anthony, Baltimore, MD 21210 (US); KALEKO, Michael, Rockville, MD 20854 (US)
(74) Representative: Brasnett, Adrian Hugh
(86) International application number: PCT/EP2003/009497
(87) International publication number: WO 2004/020469

(56) References cited:
- WO-A-01/93897
- WO-A-02/30982
- US-B1- 6 267 954
- TAKAHASHI KYOICHI ET AL: "Intraocular expression of endostatin reduces VEGF-induced retinal vascular permeability, neovascularization, and retinal detachment." FASEB JOURNAL, vol. 17, no. 8, May 2003 (2003-05), pages 896-898, XP002267463 ISSN: 0892-6638 (ISSN print)
- OHNO-MATSUI KYOKO ET AL: "Inducible expression of vascular endothelial growth factor in adult mice causes severe proliferative retinopathy and retinal detachment" AMERICAN JOURNAL OF PATHOLOGY, vol. 160, no. 2, February 2002 (2002-02), pages 711-719, XP002267464 ISSN: 0002-9440 cited in the application
- MORI KEISUKE ET AL: "Inhibition of choroidal neovascularization by intravenous injection of adenoviral vectors expressing secretable endostatin" AMERICAN JOURNAL OF PATHOLOGY, PHILADELPHIA, PA, US, vol. 159, no. 1, July 2001 (2001-07), pages 313-320, XP002205626 ISSN: 0002-9440
- OZAKI H ET AL: "Blockade of vascular endothelial cell growth factor receptor signaling is sufficient to completely prevent retinal neovascularization." AMERICAN JOURNAL OF PATHOLOGY. UNITED STATES FEB 2000, vol. 156, no. 2, February 2000 (2000-02), pages 697-707, XP002267465 ISSN: 0002-9440

## Description

### FIELD OF THE INVENTION

The present invention relates to endostatin for use in a method of treatment of retinal edema or increased retinal vascular permeability in an individual afflicted therewith. This invention also relates to use of vectors, more particularly, to retroviral vectors which may be employed for such use.

WO02/30982 relates to compositions comprising peptides or proteins that are related to endostatin for the modulation of angiogenesis. Ohno-Matsui et al (American Journal Of Pathology, Vol. 160, pages 711-719 describes inducible expression of vascular endothellal growth factor in adult mice causes severe proliferative retinopathy and retinal detachment. Mori et al (American Journal Of Pathology, Vol.159, pages 313-320) relates to inhibition of choroidal neovascularization by intravenous injection of adenoviral vectors expressing secretable endostatin. US-B1-6 267 954 describes microencapsuled cells that secrete endostatin polypeptide.

### SUMMARY OF THE INVENTION

The present invention relates to endostatin for use in a method of treatment of retinal edema or increased retinal vascular permeability in an individual afflicted therewith.

In a preferred aspect, endostatin is endostatin or an active fragment of endostatin. In another preferred aspect, the endostatin employed in the invention is a polypeptide with the amino acid sequence set forth in SEQ ID NO:1. In another preferred aspect, the endostatin is a polypeptide fragment of the polypeptide with the amino acid sequence set forth in SEQ ID NO: 1, a derivative of the polypeptide with the amino acid sequence set forth in SEQ ID NO:1, or a variant of the polypeptide with the amino acid sequence set forth in SEQ ID NO:1. Examples of such active fragments and variants are set forth, e.g., in U.S. Patent 6,174,861.

The invention is further defined In the accompanying claims.

### DETAILED DESCRIPTION OF THE INVENTION

Endostatin is a cleavage product of collagen type XVII that has been found to inhibit tumor angiogenesls and growth. Interferon α2ₐ also blocks tumor angiogenesis and causes regression of hemangiomas, but has no effect on choroidal neovascularization (CNV). The present inventors have surprisingly and unexpectedly discovered that increasing the in vivo concentration of endostatin in the ocular tissues of an individual can treat retinal edema or increased retinal vascular permeability.

In the description below there are described methods for the prophylactic and therapeutic treatment of retinal edema or increased retinal vascular permeability. The invention provides endostatin for use in such methods, in an individual afflicted therewith. "Treatment" encompasses both prophylactic and therapeutic treatment. By "prophylactic" is meant the protection, in whole or In part, against retinal edema or increased retinal vascular permeability. By "therapeutic" is meant the amelioration of retinal edema or increased retinal vascular permeability, and the protection, in whole or in part, against further retinal disorders or exacerbation of an existing disorder.

As used herein, "a retinal disorder inhibiting effective amount" of an endostatin is that amount of an endostatin that will cause any or all of: 1) a decrease in retinal vascular permeability; 2) a decrease in retinal thickness; or 3) an absolute inhibition of or a decrease in the degree of retinal detachment.

The term "DNA sequence encoding endostatin" as used herein means DNA which encodes a full-length endostatin or an active fragment, derivative, or analog of endostatin, e.g., such DNA may be a full-length gene encoding a full-length endostatin, or a truncated gene, or a mutated gene encoding a fragment or derivative or analog of such endostatin which has endostatin activity. The term "DNA sequence" refers generally to a polydeoxyribonucleotide molecule and more specifically to a linear series of deoxyribonucleotides connected one to the other by phosphodiester bonds between the 3' and 5' carbons of the adjacent pentoses.

Thus, in one embodiment, the invention is directed to a DNA sequence that encodes endostatin or a fragment, derivative, or analog thereof having endostatin activity.

DNA sequences encoding endostatin and fragments or derivatives thereof are shown and described in U.S. Pat. No. 5,854,205.

The term "endostatin" refers to a protein that is preferably 18 kDa to 20 kDa in size as determined by non-reduced and reduced gel electrophoresis, respectively. The term endostatin also includes active precursor forms of the 18 kDa to 20 kDa protein. The amino acid sequence of full-length human endostatin is set forth in SEQ ID NO:1. The nucleic acid sequence encoding human endostatin is set forth in SEQ ID NO:2. The amino acid sequence of mouse endostatin, plus the mouse lg kappa leader sequence, is set forth in SEQ ID NO:3. The nucleic acid sequence encoding mouse endostatin with the mouse lg kappa leader sequence is set forth in SEQ ID NO:4.

The term endostatin also includes fragments of the 18 kDa to 20 kDa protein and modified proteins and peptides that have a substantially similar amino acid sequence, and which are capable inhibiting proliferation of endothelial cells. For example, silent substitutions of amino acids, where the replacement of an amino acid with a structurally or chemically similar amino acid does not significantly alter the structure, conformation or activity of the protein, is well known in the art. Such silent substitutions are intended to fall within the scope of the appended claims.

It will be appreciated that the term "endostatin" includes shortened polypeptides where one or more amino acid is removed from either or both ends of full-length endostatin (i.e., the polypeptide with SEQ ID NO:1), or from an internal region of the protein, yet the resulting molecule remains effective to inhibit endothelial cell proliferation and /or to treat retinal detachment, retinal edema, and/or ocular neovascularization. Such shortened polypeptides are referred to herein as "fragments." The term "endostatin" also includes lengthened proteins or peptides where one or more amino acid is added to either or both ends of endostatin, or to an internal location in the protein, yet the resulting molecule retains endothelial proliferation inhibiting activity. Such molecules, for example with tyrosine added in the first position, are useful for labeling, using, e.g., ¹²⁵J. Labeling with other radioisotopes may be useful in providing a molecular tool for destroying the target cell containing endostatin receptors. Labeling with "targeting" molecules such as ricin may provide a mechanism for destroying cells with endostatin receptors. Lengthened endostatin polypeptides, or endostatin polypeptides that have been covalently modified, are collectively referred to herein as "derivatives" of endostatin.

"Substantial sequence homology" means at least approximately 70% homology between amino acid residue sequence in the endostatin analog sequence and that of endostatin, preferably at least approximately 80% homology, more preferably at least approximately 90% homology.

Also included in the definition of the term endostatin are modifications of the endostatin protein and its peptide fragments. Such modifications include substitutions of naturally occurring amino acids at specific sites with other molecules, including but not limited to naturally and non-naturally occurring amino acids. Such substitutions may modify the bioactivity of endostatin and produce biological or pharmacological agonists or antagonists. Such modified polypeptides are referred to herein as "variants." Variants, derivatives, and fragments of endostatin that have been shown to have antitumor effects and/or antiangiogenic effects are known and have been reported, e.g., in published international patent application numbers WO0067771, WO0063249, WO9931616, WO9929855, and WO09948924. Such variants, derivatives, and fragments of endostatin are also useful in the methods of the present invention.

The polypeptides useful in the practice of the present invention can be delivered to an individual in need of treatment using conventional pharmaceutical formulations.

An additional embodiment of the invention relates to the administration of a pharmaceutical composition, in conjunction with a pharmaceutically acceptable carrier, for any of the therapeutic effects discussed above. Such pharmaceutical compositions may consist of endostatin, e.g., endostatin, or anti-idiotypic antibodies to endostatin, or mimetics of endostatin. The compositions may be administered alone or in combination with at least one other agent, such as stabilizing compound, which may be administered in any sterile, biocompatible pharmaceutical carrier, including, but not limited to, saline, buffered saline, dextrose, and water. The compositions may be administered to a patient alone, or in combination with other agents, drugs or hormones.

The pharmaceutical compositions encompassed by the invention may be administered by any number of routes including, but not limited to, intraocular, periocular, oral, intravenous, intramuscular, intra-articular, intra-arterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated m aqueous solutions, preferably in physiologically compatible buffers such as Hanks' solution, Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers may also be used for delivery. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1-50 mM histidine, 0.1 %-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. For administration of endostatin, such labeling would include amount, frequency, and method of administration.

Pharmaceutical compositions suitable for use in the invention include compositions where the active ingredients are contained in an effective amount to achieve the intended purpose. The determination of an effective dose is well within the capability of those skilled in the art.

A therapeutically effective dose of active agent can be estimated initially either in cell culture assays, e.g., of endothelial cells, or in non-human animal models, usually mice, rabbits, dogs, or pigs. The animal model may also be used to determine the appropriate concentration range and route of administration. Such information can then be used to determine useful doses and routes for administration in humans.

A therapeutically effective dose refers to that amount of active ingredient, for example endostatin or fragments thereof, antibodies to endostatin, agonists, antagonists or inhibitors of endostatin, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio between toxic and therapeutic effects is the therapeutic index, and it can be expressed as the ratio, LD50/ED50. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED50 with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

Normal dosage amounts, e.g., when exogenously produced endostatin is administered, may vary from about 0.1 to about 20 mg/kg per day, preferably from 2.5 to 20 mg/kg per day, depending upon the route and method of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. Various biodegradable and biocompatible polymeric matrices, including microcapsules, nanospheres, and implants, are useful in the practice of the present invention.

Microspheres are fine spherical particles containing active drugs. They are differentiated from nanospheres primarily by the size of the particle; microspheres have a diameter of less than approximately 1000 µm, while nanospheres are submicronic (<1 µm). Microsphere systems contain either homogeneous monolithic microspheres, in which the drug is dissolved or dispersed homogeneously throughout the polymer matrix, or reservoir-type microspheres, in which the drug is surrounded by the polymer matrix membrane shell.

Monolithic and reservoir systems can also be combined. For instance, active drug can be dispersed within, or adsorbed onto, the polymer surface in a reservoir-type microsphere.

Biodegradable polymers can consist of either natural or synthetic materials that vary in purity. Natural polymers include polypeptides and proteins (e.g., albumin, fibrinogen, gelatin, collagen), polysaccharides (e.g., hyaluronic acid, starch, chitosan), and virus envelopes and living cells (e.g., erythrocytes, fibroblasts, myoblasts). Natural materials require cross-linking in the microencapsulation process, leading to the denaturation of the polymer and the embedded drug. As a result, synthetic polymers are most commonly used. Frequently used synthetic polymers include poly(-hydroxy) acids such as polylactic acid (PLA), polyhydroxybutryic acid, and copoly (lactic/glycolic) acid (PLGA). These compounds are biocompatible, lack immunogenicity, and have physical properties that permit them to be easily shaped (to control the bioerosion rate).

Colloidal particulate carriers can also be used in the methods of the present invention for delivering endostatin. Liposomes are the preferred colloidal vehicle, and are composed of a phospholipid bilayer that may act as a carrier for both hydrophilic and hydrophobic medications. Liposomes can be made from, e.g., neutral lipids, charged phospholipids, and cholesterol. The addition of an amphophilic polymer such as polyethylene glycol (PEG) onto the surface of a liposome can slow the clearance of liposomes.

Administration of endostatin modified with PEG is also within the scope of the present invention. PEGs are polymers comprised of repeating ethylene oxide subunits with two terminal hydroxyl groups that can be chemically activated. PEG molecules come in a number of different configurations. PEG chains include linear and branched structures in which one or more PEG chains are joined with linkers such as lysine or triazine. PEGs may be attached, preferably covalently, to endostatin at a single site or at multiple sites. Since branched-chain PEGs attach at single or fewer sites than do linear PEGs, branched PEGs may be less likely to interfere with the biologic activity of the native molecule than would the attachment of multiple small linear-chain PEGs, and so are preferred. Pharmaceutical formulations suitable for oral administration of proteins are described, e.g., in U.S. Patents 5,008,114; 5,505,962; 5,641,515; 5,681,811; 5,700,486; 5,766,633; 5,792,451; 5,853,748; 5,972,387; 5,976,569; and 6,051,561.

Endostatin can be delivered to an individual in need of treatment using gene therapy methods. Any of the methods for gene therapy available in the art can be used according to the present invention. Exemplary methods are described below.

In a preferred aspect, the therapeutic comprises endostatin-encoding nucleic acid that is part of an expression vector that expresses endostatin or fragment or chimeric protein thereof in a suitable host. In particular, such a nucleic acid has a promoter operably linked to the polypeptide coding region, the promoter being inducible or constitutive, and, optionally, tissue-specific. In another particular embodiment, a nucleic acid molecule is used in which the polypeptide coding sequences and any other desired sequences are flanked by regions that promote homologous recombination at a desired site in the genome, thus providing for intrachromosomal expression of the desired nucleic acid.

Delivery of endostatin-encoding nucleic acid into a patient may be either direct, in which case the patient is directly exposed to the nucleic acid or nucleic acid-carrying vector, or indirect, in which case, cells are first transformed with the nucleic acid in vitro, then transplanted into the patient. These two approaches are known, respectively, as in vivo or ex vivo gene therapy.

In a specific embodiment, endostatin-encoding nucleic acid is directly administered in vivo, where it is expressed to produce the encoded product. This can be accomplished by any of numerous methods known in the art, e.g., by constructing it as part of an appropriate nucleic acid expression vector and administering it so that it becomes intracellular, e.g., by infection using a defective or attenuated retroviral or other viral vector (see, e.g., U.S. Pat. No. 4,980,286 and others mentioned infra), or by direct injection of naked DNA (see, e.g., Blezinger et al., Nature Biotechnology, 17, 343-348 (1999)) or by use of microparticle bombardment (e.g., a gene gun; Biolistic, Dupont), or coating with lipids or cell-surface receptors or transfecting agents, encapsulation in liposomes (see, e.g., Chen et al., Cancer Research, 59, 3308-3312 (1999)), microparticles, or microcapsules, or by administering it in linkage to a peptide which is known to enter the nucleus, by administering it in linkage to a ligand subject to receptor-mediated endocytosis (see e.g., U.S. Patents 5,166,320; 5,728,399; 5,874,297; and 6,030,954, (which can be used to target cell types specifically expressing the receptors), etc. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO92/20316; WO93/14188; and WO 93/20221). Alternatively, the nucleic acid can be introduced intracellularly and incorporated within host cell DNA for expression, by homologous recombination (see, e.g., U.S. Patents 5,413,923; 5,416,260; and 5,574,205.

In a specific embodiment, a viral vector that contains endostatin-encoding nucleic acid is used. For example, a retroviral vector can be used (see, e.g., U.S. Patents 5,219,740; 5,604,090; and 5,834,182). These retroviral vectors have been modified to delete retroviral sequences that are not necessary for packaging of the viral genome and integration into host cell DNA. Endostatin-encoding nucleic acid to be used in gene therapy is cloned into the vector, which facilitates delivery of the gene into a patient.

Adenoviruses are another type of viral vector that can be used in gene therapy. Adenovirus genomes are linear, double-stranded DNA molecules of approximately 36 kilobase pairs. Each extremity of the viral genome has a short sequence known as the inverted terminal repeat (or ITR), which is necessary for viral replication. The well-characterized molecular genetics of adenovirus render it an advantageous vector for gene transfer. Portions of the viral genome can be substituted with DNA of foreign origin. In addition, recombinant adenoviruses are structurally stable and no rearranged viruses are observed after extensive amplification.

Adenoviruses are especially attractive vehicles for delivering genes to respiratory epithelia. Adenoviruses naturally infect respiratory epithelia where they cause a mild disease. Other targets for adenovirus-based delivery systems are liver cells, the central nervous system, endothelial cells, and muscle. Adenoviruses have the advantage of being capable of infecting non-dividing cells. Methods for conducting adenovirus-based gene therapy are described in, e.g., U.S. Patents 5,824,544; 5,868,040; 5,871,722; 5,880,102; 5,882,877; 5,885,808; 5,932,210; 5,981,225; 5,994,106; 5,994,132; 5,994,134; 6,001,557; and 6,033,8843.

In a specific embodiment, the nucleic acid to be introduced for purposes of gene therapy using an adenoviral vector comprises an inducible promoter operably linked to the coding region, such that expression of the nucleic acid is controllable by controlling the presence or absence of the appropriate inducer of transcription.

The incorporation of genomic elements into the adenoviral vector may provide for enhanced expression of the DNA sequence encoding endostatin. Thus, in accordance with another aspect of the present invention, there is provided an adenoviral vector including at least one DNA sequence encoding a endostatin, and at least one genomic element affecting the expression of such DNA sequence. The term "genomic element" is used as previously defined. Such genomic elements include, but are not limited to, introns, the 5' untranslated region, and the 3' untranslated region, and portions of the introns and 3' and 5' untranslated regions. The adenoviral vector may be as hereinabove described. Promoters which control the DNA sequence may be selected from those described herein and from those known in the art.

The vector, consisting of infectious, but replication-defective, viral particles, which contain at least one DNA sequence encoding endostatin, is administered in vivo to a host in an amount effective to treat choroidal neovascularization in the host. The host may be a mammalian host, including human and non-human primate hosts.

The adenoviral vector may be administered to a mammalian host in an amount effective to provide endostatin levels of up to 1,000,000 ng/ml of blood or 1 mg/ml of blood. Although the adenoviral vector may be administered to a mammalian host in an amount effective to provide endostatin levels of up to 1,000,000 ng/ml, it has been found that some endostatin, such as endostatin, when expressed by mammalian cells transduced with an adenoviral vector of the present invention, is significantly more active (about 1,000 times more active) than endostatin expressed by non-mammalian cells, such as yeast cells or bacterial cells such as E. coli cells. Thus, in order to achieve a desired anti-neovascularization effect, one can generally provide a mammalian host with endostatin at lower levels by administering the adenoviral vector to a mammalian host, as opposed to providing a mammal with significantly greater levels of endostatin expressed by yeast or bacteria.

In one embodiment, when administered to a mammalian host, the adenoviral vector is administered in an amount effective to provide endostatin levels which are from about 2 to 20 times the basal levels of endostatin found in the host. In general, in such an embodiment, the adenoviral vector is administered to a mammalian host in an amount effective to provide for expression of the active polypeptide at a level of at least about 300 ng/ml of blood, preferably from about 300 ng/ml to about 3000 ng/ml, more preferably from about 500 ng/ml to about 1500 ng/ml.

In another embodiment, a viral vector is administered in an amount of from about 10⁸ plaque forming units to about 10¹⁴ plaque forming units, preferably from about 10⁸ plaque forming units to about 10¹¹ plaque forming units, more preferably from about 10⁹ plaque forming units to about 10¹⁰ plaque forming units. Adenoviral vectors, in the quantities set out above, are preferred.

The infectious vector particles can be administered systemically, such as, for example, by intravenous administration (such as, for example, via peripheral vein injection) or administered via the portal vein, to the bile duct, intramuscularly, intraperitoneally, or intranasally. Alternatively, infectious vector particles can be administered locally, by, e.g, intraocular or periocular injection. Such injection can be either into the anterior or posterior chamber of the eye, e.g., into the aqueous humor or vitreous humor. Alternatively, the injection can be subretinal, e.g., by injection of aliquots (e.g., 1 to 10 microliters per aliquot) of vector-containing solution behind the retina, after which the solution is absorbed and the infectious vector particles infect local cells of the ocular tissues and produce the active polypeptide. Such administration can comprise either a single injection, multiple injections administered on the same day, single injections administered over a period of weeks or months, or multiple injections administered over a period of weeks or months.

The vector particles may be administered in combination with a pharmaceutically acceptable carrier suitable for administration to a patient. The carrier may be a liquid carrier (for example, a saline solution), or a solid carrier, such as, for example, mirocarrier beads.

Adeno-associated virus (AAV) has also been proposed for use in gene therapy, including endostatin gene therapy for tumors (see, e.g., Nguyen et al., Cancer Research, 58, 5673-5677 (1998)). Methods for producing and utilizing AAV are described, e.g., in U.S. Patents 5,173,414; 5,252,479; 5,552,311; 5,658,785; 5,763,416; 5,773,289; 5,843,742; 5,869,040; 5,942,496; and 5,948,675.

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny. In another embodiment, an endogenous gene in a cell that is normally not expressed, or expressed at a low level by the cell, can be activated by the operatively linking a strong promoter to the endogenous gene, thus providing a cell that expresses an endogenous gene at high levels, leading to the synthesis and secretion of endostatin by the cell.

Methods for the production and administration of such cells (i.e., those that produce high levels of protein from either endogenous or exogenous genes or nucleic acids) are described in, inter alia, U.S. Patent Nos. 5,641,670; 5,733,761; 5,968,502; 6,048,729; 6,054,288; 6,063,630; and 6,187,305. In a preferred embodiment, endostatin-producing cells are delivered in microencapsulated form, e.g., in the form of cells microencapsulated in sodium alginate or calcium alginate poly L-lysine alginate (see, e.g., Read et al., Nature Biotechnology 19, 29-34 (Jan 2001) and Joki et al., Nature Biotechnology 19, 35-39 (Jan 2001)). The microencapsulated cells can be implanted proximally to the eye or at a site where the endostatin produced by the cells will most quickly enter the subject's bloodstream, e.g., in the liver. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, an endostatin-encoding nucleic acid is introduced into the cells such that it is expressible by the cells or their progeny, and the recombinant cells are then administered in vivo for therapeutic effect. In a specific embodiment, stem or progenitor cells are used. Any stem-and/or progenitor cells which can be isolated and maintained in vitro can potentially be used in accordance with this embodiment of the present invention. Such stem cells include but are not limited to hematopoietic stem cells (HSC), stem cells of epithelial tissues such as the skin and the lining of the gut, non-human embryonic heart muscle cells, liver stem cells (see, e.g., WO 94/08598), and neural stem cells.

Epithelial stem cells (ESCs) or keratinocytes can be obtained from tissues such as the skin and the lining of the gut by known procedures. In stratified epithelial tissue such as the skin, renewal occurs by mitosis of stem cells within the germinal layer, the layer closest to the basal lamina. Stem cells within the lining of the gut provide for a rapid renewal rate of this tissue. ESCs or keratinocytes obtained from the skin or lining of the gut of a patient or donor can be grown in tissue culture. If the ESCs are provided by a donor, a method for suppression of host versus graft reactivity (e.g., irradiation, drug or antibody administration to promote moderate immunosuppression) can also be used.

With respect to hematopoietic stem cells (HSC), any technique which provides for the isolation, propagation, and maintenance in vitro of HSC can be used in this embodiment of the invention. Techniques by which this may be accomplished include (a) the isolation and establishment of HSC cultures from bone marrow cells isolated from the future host, or a donor, or (b) the use of previously established long-term HSC cultures, which may be allogeneic or xenogeneic. Non-autologous HSC are used preferably in conjunction with a method of suppressing transplantation immune reactions of the future host/patient. In a particular embodiment of the present invention, human bone marrow cells can be obtained from the posterior iliac crest by needle aspiration (see, e.g., Kodo et al., 1984, J. Clin. Invest. 73:1377-1384). HSCs can be made highly enriched or in substantially pure form. This enrichment can be accomplished before, during, or after long-term culturing, and can be done by any techniques known in the art. Long-term cultures of bone marrow cells can be established and maintained by using, for example, modified Dexter cell culture techniques (Dexter et al., 1977, J. Cell Physiol. 91:335) or Witlock-Witte culture techniques (Witlock and Witte, 1982, Proc. Natl. Acad. Sci. USA 79:3608-3612).

It is understood, however, that the scope of the present invention is not to be limited to the specific embodiments described above. The invention may be practiced other than as particularly described and still be within the scope of the accompanying claims.

A still further aspect is pertaining to the use of an endostatin in the manufacture of a medicament for the treatment of retinal detachment or retinal edema in an individual afflicted therewith, wherein said endostatin is in particular a polypeptide with the amino acid sequence set forth in SEQ ID NO:1., or wherein said endostatin is as defined and described throughout this disclosures.

### EXAMPLE 1

### Generation of Adenoviral Vectors: Method 1

The mouse endostatin (mEndo) cDNA is amplified by polymerase chain reaction (PCR) from mouse collagen XVIII clone ID 748987 from Genome Systems (St. Louis, MO) with the primers 5'-ACT GGT GAC GCG GCC CAT ACT CAT CAG GAC TTT CAG CC-3' (SEO ID NO:6) and 5'-AAG GGC TAT CGA TCT AGC TGG CAG AGG CCT AT-3' (SEQ ID NO:7) (598-bp F1 fragment). The mouse immunoglobulin k chain leader sequence (lg-k leader) is PCR amplified from pSecTag2 (InVitrogen, Carlsbad, CA) with the primers 5'-CAC TGC TTA CTG GCT TAT CG-3' (SEQ ID NO:8)and 5'-CTG ATG AGT ATG GGC CGC GTC ACC AGT GG-3' (SEQ ID NO:9) (147-bp F2 fragment). PCR is carried out with Pfu DNA polymerase (Stratagene, La Jolla, CA) for 35 cycles under the following conditions: 95°C hot start for 3 min, 95°C denaturation for 1 min, 55°C annealing for 1 min, and 72°C extension for 2 min. The DNA fragments are gel purified. The sig-mEndo chimeric DNA (718 bp) is generated by PCR splice overlap extension with F1 and F2 DNA fragments generated above as templates to assemble mouse lg-k leader sequence and murine endostatin cDNA. PCR is carried out with the primers 5'-CAC TGC TTA CTG GCT TAT CG-3' (SEQ ID NO:8) and 5'-AAG GGC TAT CGA TCT AGC TGG CAG AGG CCT AT-3' (SEQ ID NO:10), using Pfu DNA polymerase (Stratagene). PCR is run for 35 cycles under the following conditions: 95°C hot start for 3 min, 95°C denaturation for 1 min, 60°C annealing for 1 min, and 72°C extension for 2 min.

The pAvmEndoLxr adenoviral shuttle plasmid is constructed by inserting the 718-bp sig-mEndo chimeric DNA into the NheI and Clal sites of adenoviral shuttle plasmid, pAvF9lxr, which is downstream of the Rous sarcoma virus (RSV) promoter and upstream of the simian virus 40 (SV40) polyadenylation signal. An AscI and Nhe1 digested simian cytomegalovirus (sCMV) promoter fragment is substituted for the RSV promoter in pAvmEndoLxc, which is otherwise identical to pAvmEndoLxr. Both shuttle plasmids contain a LoxP site for Cre/lox-mediated recombination. The sequence of the transgenes in the pAvmEndoLxr and pAvmEndoLxc adenoviral plasmids are confirmed by direct sequencing analysis.

Recombinant Av3mEndo (with E1, E2a, and E3 deleted) encoding the sig-mEndo chimera is generated by Cre/lox-mediated recombination of two plasmids, pSQ3 and pAvmEndoLxr. The pSQ3 plasmid contains a loxP site followed by the Av3 genome with the deletion of the region from the left-end inverted terminal repeat (ITR) to the end of E1a. pAvmEndoLxr and pSQ3 are first linearized with Notl and ClaI restriction enzymes, respectively. A transient transfection is performed with 293 cells (4x10⁵ cells per well of a six-well plate), using the calcium phosphate mammalian transfection system (Promega, Madison, WI). The calcium phosphate-DNA precipitate is prepared with 4.8 mg of linearized pAvmEndoLxr, 12 mg of linearized pSQ3, 6mg of pcmvCre, and 6 mg of pcmvE2a in a total volume of 1.8 ml. A 0.6-ml calcium phosphate-DNA precipitate is added to each well. The 293 cells are incubated with calcium phosphate-DNA precipitate at 37°C for 16 hr. The precipitate is removed and the cells are washed with phosphate-buffered saline (PBS). Fifteen days posttransfection, cytopathic effect (CPE) is observed. The cells and the medium are then harvested by scraping. The crude viral lysate is prepared by five cycles of freezing and thawing.

The Av3mEndo vector is reamplified in S8 cells with 0.3 mM dexamethasone in Richter's CM containing 5% FBS until CPE is observed. The adenoviral vector titer (particles per milliliter) and biological titer (plaque-forming units [PFU] per milliliter) are determined as described (Mittereder et al., 1996). Recombinant Av3CsmEndo containing sig-mEndo driven by the CMV promoter is generated in the same manner by Cre/lox-mediated recombination of pSQ3 and pAvmEndoLxc. The correct genome structures of the purified Av3mEndo, Av3CsmEndo, and control Av3Null are confirmed by restriction digests and Southern blot analysis. The Av3mEndo and Av3CsmEndo seedlot are confirmed to be negative for replication-competent adenovirus (RCA).

The supernatant from Av3mEndo-transformed S8 cells contains a 20-kDa protein, the expected size of endostatin, that potently inhibits VEGF165-induced migration of HUVEC cells and ELISA demonstrated that 10⁶ Av3mEndo-transduced Hep3B cells secrete 1-2 µg of murine endostatin per 24 hours.

### Generation of Adenoviral Vectors: Method 2

Murine cDNA is obtained by isolating RNA (RNeasy Mini kit; Qiagen, Valencia, CA) from snap-frozen 2-week-old C57BU6 mouse (Charles River Laboratories, Wilmington, MA) liver and by treating with Moloney murine leukemia virus reverse transcriptase (Life Technologies, Inc., Gaithersburg, MD). The murine endostatin gene is cloned into the TA cloning vector (Invitrogen, Carlsbad, CA) by PCR using the primers sense 5'-GATCTCTAGACCACCATGCATACTCATCAGGACTT-3' (SEQ ID NO:11) and antisense 5'-ACTGGAGAAAGAGGTTTATCTAGCTACTAG-3' (SEQ ID NO:12). The 18-amino acid E3/19K signal sequence MRYMILGLLALAAVCSAA (SEQ ID NO:13) is inserted upstream from the endostatin sequence by PCR using the primers sense 5'-GATCTCTAGACCACCATGAGGTACATGATTTTAGGCTTGCTCGCCCTTGCGG CAGTCTGCAGCGCGGCCCATACTCATACTCATCAGGACTTTCAG-3' (SEQ ID NO:14) and antisense (as above). Plasmid DNA is amplified in DH5 cells (Life Technologies), and the signal sequence-murine endostatin (ss-mEndo) sequence is confirmed (ABI Prism 310 autosequencer; PE Applied Biosystems, Foster City, CA).

The ss-mEndo construct is digested with EcoRI and cloned by blunt-end ligation into the multiple cloning site of the adenoviral shuttle plasmid pAd/CMV.1. The resulting plasmid is recombined with type 5 E1A/B-deleted Ad2 and used to infect 293 cells (American Type Culture Collection, Manassas, VA). Plaque DNA is extracted using proteinase K digestion, phenol extraction, and ethanol precipitation and screened for ss-mEndo by PCR. The resulting virus, Ad-ss-mEndo, is amplified in 293 cells. A similar strategy is used to create control recombinant viruses containing the genes for β-gal (Ad-β-gal) and firefly luciferase (Ad-luc). Viruses are titered using a standard plaque-forming assay in 293 cells. Cells are grown in complete medium consisting of DMEM with 10% FCS, 100 units/ml penicillin, 100 µg/ml streptomycin, 50 µg/ml gentamicin, 0.5 µg/ml Fungizone, and 4 mM glutamine (Biofluids, Rockville, MD). Cells are infected at MOIs ranging from 0.1 to 100 (105 to 108 pfu per 106 cells in 1.0 ml of complete media) with Ad-ss-mEndo, Ad-luc, or no virus and incubated at 37°C for 24 h. Supernatants are centrifuged at 2 x g for 5 min and assayed for endostatin using a competitive EIA (Cytimmune Sciences, College Park, MD), according to the manufacturer's instructions. 293 cell supernatants are concentrated 10-fold in cellulose columns (Centricon YM-10; Millipore, Bedford, MA) and analyzed by Western blotting (NuPAGE; Novex, San Diego, CA) using 570 ng/ml rabbit antimurine endostatin polyclonal IgG antibody (gift of Cytimmune Sciences). The EIA murine endostatin standard is used as a positive control. The susceptibility of the murine colon adenocarcinoma cell line MC38 (developed in the Surgery Branch, National Cancer Institute) to adenoviral infection is tested by infecting cells with Ad-β-gal as described above and assaying for β-gal 24 h later using a staining kit (Boehringer Mannheim, Indianapolis, IN). Susceptibility of the murine hepatocyte line NMuLi (American Type Culture Collection) to Ad-β-gal infection is used as a positive control.

### Generation of Adenoviral Vectors: Method 3

Liver tissue from a BALB/c mouse is homogenized, and total RNA is extracted (RNeasy kit; Qiagen, Chatsworth, CA). First-strand cDNA is amplified by reverse transcription-PCR with oligo(dT) primers (SuperScript II; Life Technologies, Grand Island, NY). The full-length mouse endostatin cDNA is amplified by PCR (sense primer with a Clal linker, 5'-ATCGATCATACTCATCAGGACTTTCAGCC-3' (SEQ ID NO:15); antisense primer with a Notl linker, 5'-GCGGCCGCCTATTTGGAGAAAGAGGTCAT-3' (SEQ ID NO:16) for subcloning into pBluescript (Stratagene). A synthetic oligonucleotide coding for the rat insulin leader sequence is cloned in front of the endostatin gene. After sequence confirmation, the rat insulin leader-endostatin cDNA is cloned into the recombinant adenovirus (ADV) shuttle vector pADV.hEF1-α (human elongation factor 1-α) for the rescue of the recombinant adenovirus as described by Bautista, D.S. et al., (1991) Virology 182, 578-596. The viral particles are measured by absorption (A260), and the plaque-forming units are determined by standard agarose-overlay plaque assay on 293 cells. The cDNA for the construction of the ADV.hVEGF165 is obtained through reverse transcription-PCR of RNA isolated from human umbilical vein endothelial cells (HUVEC). JC and LLC cell lines are obtained from American Type Culture Collection. The cells are cultured in RPMI medium 1640 (JC) and DMEM (LLC). All media are supplemented with 10% FBS, 0.2 mM glutamine, and 1% penicillin/streptomycin. HUVEC are isolated from umbilical cords by collagenase type IV (Sigma) perfusion (0.2% in Hanks' balanced salt solution) for 20 min at room temperature. The cells then are cultured on collagen-coated (1% in PBS) plates in M199 medium supplemented with 20% FBS, 0.2 mM glutamine, 1% penicillin/streptomycin, and 1 ng/ml bFGF.

### EXAMPLE 2: GENE TRANSFER TO MICE AND INDUCTION OF CNV

Viral vectors are injected into the tail vein of adult C57BU6 mice. Mice are injected with 2x10¹¹ particles of either Av3mEndo (n=18) or Av3mNull (n=17) or with 6x10¹⁰ particles of either Av3CsmEndo or Av3CsNull. Four days after viral vector injection, the mice are anesthetized with ketamine hydrochloride (100 mg/kg body weight), pupils are dilated with 1% tropicamide, and krypton laser photocoagulation is used to rupture Bruch's membrane at 3 locations in each eye of each mouse as previously described by Tobe, et al. Am. J. Pathol. 153, 1641-1646 (1998). Briefly, krypton laser photocoagulation (100 µm spot size, 0.1 seconds duration, 120 mW) is delivered using the slit lamp delivery system of a Coherent Model 920 Photocoagulator and a hand held cover slide as a contact lens. Burns are performed in the 9,12, and 3 o'clock positions 2-3 disc diameters from the optic nerve. Production of a vaporization bubble at the time of laser, which indicates rupture of Bruch's membrane, is an important factor in obtaining CNV, so only burns in which a bubble is produced are included in the study. A bubble is not produced for 1 burn in mice injected with Av3mEndo and 3 burns in mice injected with Av3mNull. The cornea of one eye of a mouse that had been injected with Av3mEndo has a corneal scar that prevented laser use and that eye is not used.

### EXAMPLE 3: MEASUREMENT OF THE SIZE OF LASER-INDUCED CNV LESIONS

Two weeks after laser treatment, the size of CNV lesions is evaluated by one of two different techniques, measurement of the integrated area of CNV on serial sections as previously reported by Seo, et al., Amer. J. Pathol. 154, 1743-1753 (1999) or measurement of the area of CNV in choroidal flat mounts as described by Edelman et al., Invest. Ophthalmol. Vis. Sci. 41, S834 (2000). For mice injected with Av3mEndo, 10 mice are evaluated by the flat mount technique and 8 by serial sections, and for mice injected with Av3mNull, 10 mice are evaluated by the flat mount technique and 7 by serial sections.

Mice used for the flat mount technique are anesthetized and perfused with 1 ml of phosphate-buffered saline containing 50 mg/ml of fluorescein-labeled dextran (2x10⁶ average mw, Sigma, St. Louis, MO) as previously described by Tobe, et al., Invest. Ophthalmol. Vis. Sci. 39,180-8 (1998). The eyes are removed and fixed for 1 hour in 10% phosphate-buffered formalin. The cornea and lens are removed and the entire retina is carefully dissected from the eyecup. Radial cuts (4-7, average 5) are made from the edge of the eyecup to the equator and the eyecup is flat mounted in Aquamount with the sclera facing down and the choroid facing up. Flat mounts are examined by fluorescence microscopy and images are digitized using a 3 CCD color video camera and a frame grabber. Image-Pro Plus is used to measure the total area of hyperfluorescence associated with each burn, corresponding to the total fibrovascular scar.

For mice injected with Av3mEndo, a total of 19 eyes are evaluated (one eye had a pre-existent corneal scar that precluded laser treatment) and there is one burn that had not been associated with a bubble, so that 56 lesions are measured. For mice injected with Av3mNull, a total of 20 eyes are evaluated and since there are 3 burns that had not been associated with a bubble, 57 lesions are measured. The areas within each eye are averaged and after log transformation, regression analysis with generalized estimating equations (GEE) is performed. This analysis adjusts for correlation between right and left eyes of each mouse.

Mice used to measure the integrated area of CNV on serial sections are sacrificed 2 weeks after laser treatment and eyes are rapidly removed and frozen in optimum cutting temperature embedding compound (OCT; Miles Diagnostics, Elkhart, IN). Frozen serial sections (10 µm) are cut through the entire extent of each burn and histochemically stained with biotinylated griffonia simplicifolia lectin B4 (GSA, Vector Laboratories, Burlingame, CA) which selectively binds to vascular cells. Slides are incubated in methanol/H₂O₂ for 10 minutes at 4°C, washed with 0.05 M Tris-buffered saline, pH 7.6 (TBS), and incubated for 30 minutes in 10% normal porcine serum. Slides are incubated 2 hours at room temperature with biotinylated GSA and after rinsing with 0.05M TBS, they are incubated with avidin coupled to peroxidase (Vector Laboratories) for 45 minutes at room temperature. After being washed for 10 minutes with 0.05 M TBS, slides are incubated with Histomark Red (Kirkegaard and Perry) to give a red reaction product that is distinguishable from melanin. Some slides are counterstained with Contrast Blue (Kirkegaard and Perry).

To perform quantitative assessments, GSA-stained sections are examined with an Axioskop microscope and images are digitized using a 3 CCD color video camera and a frame grabber. Image-Pro Plus software is used to delineate and measure the area of GSA-stained blood vessels in the subretinal space. For each lesion, area measurements are made for all sections on which some of the lesion appeared and added together to give the integrated area measurement. The measurements within each eye are averaged and regression analysis with GEE is performed.

In initial experiments, the amount of CNV at sites of laser-induced rupture of Bruch's membrane is compared in mice injected with Av3mEndo and mice injected with Av3mNull. The amount of CNV is assessed by two different techniques; measurement of the area of CNV perfused by fluorescein-labeled dextran on choroidal flat mounts and measurement of the area of CNV on serial sections through the entire lesion. The area of laser-induced CNV in choroidal flat mounts appeared less in mice injected with Av3mEndo compared to uninjected mice or mice injected with Av3Null. The difference seen by visual comparison is confirmed by image analysis performed by investigators masked with respect to treatment group, which showed that the mean area of perfused CNV lesions in mice injected with Av3mEndo is significantly less than that in Av3Null-injected controls (Table 1).

**TABLE 1**

| **Area of Perfused CNV on Choriodal Flat Mounts** | | | | | | |
|---|---|---|---|---|---|---|
| Vector | | Mice | Eyes | Lesions | Area (10⁻³ mm²) | P |
| Av3mEndo | | 10 | 19 | 56 | 13.73 ± 1.36 | <0.0001 |
| Av3Null | | 10 | 20 | 57 | 29.41 ± 2.19 | |

| **Integrated Area of CNV on Serial Sections Through Entire Lesions** | | | | | | |
|---|---|---|---|---|---|---|
| Vector | Mice | Eyes | Lesions | Integrated Area (10⁻² mm²) | | P |
| Av3mEndo | 8 | 15 | 44 | 5.88 ± 0.91 | | <0.0001 |
| Av3Null | 7 | 13 | 37 | 12.58 ± 2.21 | | |

Serial sectioning through CNV lesions also showed smaller lesions in mice injected with Av3mEndo compared to mice injected with Av3Null. The integrated area of CNV obtained by adding together the area of CNV on each serial section, which assesses size in 3 dimensions, confirmed that there is significantly less CNV at sites of Bruch's membrane rupture in mice injected with Av3mEndo compared to Av3Null injected-mice (Table 1). Since both measurement techniques provide very similar information, only choroidal flat mounts are used in subsequent experiments.

There is an inverse correlation between endostatin serum levels and the area of CNV. Serum levels of endostatin are optimal 4-7 days after intravenous injection of the vectors. A group of mice are injected with Av3mEndo, Av3CsmEndo, Av3Null, or Av3CsNull. Laser treatment is done on day 4 and serum is obtained 7 days after injection. With investigators masked with respect to vector group and endostatin serum level, the area of CNV is measured on choroidal flat mounts 14 days after laser photocoagulation. Mice injected with Av3CsmEndo appear to have less CNV than uninjected mice or those injected with Av3CsNull. Image analysis confirms that the area of CNV lesions is significantly less in mice injected with either Av3CsmEndo or Av3mEndo compared to controls (Table 2).

**TABLE 2: Area of Perfused CNV on Choriodal Flat Mounts**

| Vector | Mice | Eyes | Lesions | Area (10⁻³ mm²) | P |
|---|---|---|---|---|---|
| Av3CsmEndo | 11 | 22 | 66 | 8.87 ± 0.85 | *<0.0001, **<0.0001 |
| Av3mEndo | 10 | 19 | 55 | 18.36 ± 2.24 | *0.0013, **0.0004 |
| Av3CsNull | 11 | 21 | 62 | 24.41 ± 2.92 | *0.22 |
| Av3Null | 9 | 17 | 48 | 32.91 ± 4.87 | *0.89 |
| No vector | 11 | 21 | 59 | 31.71 ± 3.98 | |

| | | | | | |
|---|---|---|---|---|---|
| * for difference from no vector controls; **for difference from coresponding null vector control | | | | | |

Plotting the mean area of CNV lesions vs. endostatin serum level in each mouse shows a strong inverse correlation with r = -0.66.

### EXAMPLE 4: ANALYSIS OF EXPRESSION OF ENDOSTATIN IN EYE AND LIVER

To determine whether systemic administration of adenoviral vectors results in significant transduction of the eye, a group of mice is injected with Av3nBg. This vector expresses β-gatactosidase from an RSV promoter. After 5 days, the mice are sacrificed and β-gatactosidase activity is measured in homogenates of the eye and liver using a chemiluminescence assay. Livers and eyes are snap frozen following removal from mice. On the day of the assay, livers or eyes are homogenized in lysis buffer (40:1 v/v 1X Reporter Lysis Buffer (Promega, Madison WI): Protease Inhibitor Cocktail (Sigma, St Louis MO)). Protein content is determined by Bradford Assay (Biorad, Hercules CA). β-galactosidase activity is determined using the Galacto-Light system (Tropix, Bedford MA).

In the livers of mice that received vector, levels of β-gatactosidase activity are approximately 1000-fold higher than uninjected controls, whereas in the eye, the levels of this enzyme activity are similar between vector-injected and control animals. The absence of detectable β-gaiactosidase activity in the eye following administration of an adenovirus expressing this enzyme suggests that the antiangiogenic effect after intravascular injection of endostatin vectors is due to systemically-produced rather than locally-produced endostatin.

### EXAMPLE 5: COMPARISON OF MICE INJECTED WITH AV3MENDO TO THOSE INJECTED WITH Av3CSMENDO

Mice are injected in the tail vein with 2X10¹¹ particles of Av3mEndo (n=10) or Av3mNull (n=9), or they are injected with 6X10¹⁰ particles of Av3CsmEndo (n=11) or Av3CsmNull (n=11). A no injection control group (n=11) is also included. Four days after injection, Bruch's membrane is ruptured with laser in three places in each eye of each mouse as described above. Seven days after injection, blood is drawn from the tail vein of each mouse and serum is stored at -80°C for ELISAs. Eighteen days after injection and 14 days after laser, the area of CNV is assessed on choroidal flat mounts as described above.

Endostatin serum levels are determined with a murine endostatin enzyme-linked immunosobent assay (ELISA) kit (ACCUCYTE murine endostatin:Cytlmmune Sciences, College Park, MD) according to the manufacturer's instructions.

Characterization of the second vector construct, Av3CsmEndo, demonstrated that its intravascular injection results in approximately 10-fold higher maximal endostatin levels compared to levels in mice that are injected with the maximum tolerated dose of Av3mEndo particles (2 x 10¹¹ pfu). Serum levels of endostatin are significantly higher in the Av3mEndo and Av3CsmEndo injected mice than in controls with no injection or a null vector injection. Basal levels of endostatin in mice are found to be between about 30 to 150 ng/ml of serum.

Thus, mice that are injected with a construct in which sig-mEndo expression is driven by the Rous sarcoma virus promoter have moderately high serum levels of endostatin and significantly smaller CNV lesions at sites of laser-induced rupture of Bruch's membrane than mice that are injected with null virus. Mice that are injected with a construct in which sig-mEndo is driven by the simian cytomegalovirus promoter have roughly 10-fold higher endostatin serum levels and have significantly less CNV, with nearly complete inhibition.

### EXAMPLE 6: GENERATION OF A RECOMBINANT ADENOVIRAL VECTOR ENCODING HUMAN ENDOSTATIN

The human endostatin cDNA is PCR amplified from the cDNA of human α1 (XVIII) collagen. The human liver cDNA is generated from human liver poly A RNA (Clonetech, Palo Alto, CA) by reverse transcriptase polymerase chain reaction (RT- PCR). The reverse transcription is carried out with the primer of 5'-TTT TTT TTT CAG TGT AAA AGG TC-3' (SEQ ID NO: 17) using the Perkin Elmer RT-PCR kit (Perkin Elmer Applied Biosystems, Foster City, CA) for 1 cycle in the following conditions: room temperature for 10 min, 42°C reverse transcribing for 3 min, 99°C denaturation for 5 min, 5°C cooling for 5 min, and hold at 4°C until the cDNA is ethanol precipitated and resuspended. The 790 bp human endostatin cDNA fragment is PCR amplified from the prepared cDNA with the primers of 5'-CAG ATG ACA TCC TGG CCA G-3' (SEQ ID NO: 18) and 5'-CTA TAC AGG AAA GTA TGG CAG C-3' (SEQ ID NO: 19). PCR is carried out for 35 cycles in the following condition: 95°C hot start for 3 min, 80°C for 3 min followed by the addition of Pfu DNA polymerase (Stratagene, La Jolla, CA), 95°C denaturation for 1 min, 55°C annealing for 1 min, and 72°C extension for 3 min. The 790 bp human endostatin cDNA fragment is gel purified and reamplified as described except using the annealing temperature of 58°C. The 790 bp human endostatin cDNA fragment is gel purified and cloned into PCR-Script Amp SK+ using PCR-Script Cloning Kits (Stratagene) according to the manufacturer's procedure to generate pcrhend I. The human endostatin cDNA region of the pcrhend I plasmid is confirmed with the direct sequencing analysis by Gene Therapy Core Technologies Molecular Core Laboratory at Genetic Therapy, Inc. Gaithersburg, MD.

The human endostatin cDNA fragment is assembled with human BM40 basement protein leader according to the following procedure. The BM40 basement protein leader is generated by annealing 2 pieces of synthesized oligonucleotides, 5'-GCC AAG CTT CCA TGA GGG CCT GGA TCT TCT TTC TCC TTT GCC TGG CCG GGA GGG CTC TGG CAG CCC CTC AGC AAG AAG CGC TCG CTC ACA GCC ACC GCG ACT TCC AGC CGG TGC TCC A-3' (sense) (SEQ ID NO:20), and 5'-CCA GGT GGA GCA CCG GCT GGA AGT CGC GGT GGC TGT GAG CGA GCG CTT CTT GCT GAG GGG CTG CCA GAG CCC TCC CGG CCA GGC AAA GGA GAA AGA AGA TCC AGG CCC TCA TGG AAG CTT GGC-3' (antisense) (SEQ ID NO:21) followed by Hind III and Sex A1 digestion. The digested BM40 basement protein leader is cloned into Hind III and Sex A1 sites of pcrhend I to generate pBmpcrhen plasmid. The entire sig-hEndo region of the pBmpcrhen plasmid is confirmed with the direct sequencing analysis.

The adenovial shuttle plasmid pAV1 bmhend1 x is generated by substitution of the Factor IX (F9) containing sequence with the sig-Endo containing sequence in pAvF9Lxr adenoviral shuttle plasmid in the following procedure. An 800 bp fragment containing sig-hEndo sequence is generated from pBmpcrhen by SacI digestion followed by Klenow fill in and Sal I digestion. The pAvF9Lxr plasmid is digested with Bam HI restriction enzyme followed by Klenow fill in and digested with Sal I restriction enzyme to remove F9 containing sequences. The two digested fragments are gel purified and ligated to generate pAV1 bmhendlx.

Human endostatin cDNA is RT-PCR generated from the C-terminus of cDNA of human α1 (XVIII) collagen from human liver poly A RNA. The human BM40 basement protein leader is generated from two pieces of synthesized oligonucleotides. The annealed human BM40 basement protein leader is cloned 5' of the human endostatin cDNA to generate sig-hEndo chimeric protein for the secretion of human endostatin protein. The sig-hEndo chimeric DNA is cloned into the adenoviral shuttle plasmid, pAvF9Ixr to create pAV1bmhendlx (Fig. 12A). The entire sig-hEndo chimeric sequence is confirmed by auto sequencing analysis.

Recombinant Av3bmhendlx (with E1, E2a, and E3-deletions) encoding the sig-hEndo chimeric protein is generated by the "Quick Cre/Lox two plasmid system" according to the following procedure. The plasmids pAV1bmhendlx and pSQ3 are linearized first with Not I and Cla I restriction enzymes, respectively. The S8 cells are pretreated with 0.3 µM dexamethasone 24 hours before the transient transfection that is performed on the 6-well plate at 4 x 105 S8 cells per well using LipofectAMINE PLUS Reagent (Life Technologies, Rockville, MD). The lipofectamine complexed DNA is prepared with 1 µg of linearized pSQ3, 0.5 µg pCre, and 0.5 µg linearized pAV1bmhendlx, and 6 µl of lipofectamine according to the manufacturer's procedure (Life Technologies). The S8 cells are incubated with lipofectamine complexed DNA at 37°C for 4.5 hours. The lipofectamine complexed DNA is removed and the cells are ished with PBS. The transfected S8 cells are cultured at 37°C with 5% CO2 until the cytopathic effect is observed. The cells and the medium are harvested by scraping. The crude viral lysate is prepared by five cycles of freezing and thawing. The Av3bmhendlx is re-amplified in S8 cells with 0.3 µM dexamethasone in Richter's CM medium containing 5% FBS until cytopathic effect is observed.

Av3bmhendlx-mediated human endostatin expression and secretion is characterized in vector-transduced S8 cells. The supernatant protein of cells infected with Av3bmhendlx, i.e., human endostatin, is analyzed by SDS-PAGE. Each 20 µg of supernatant protein is analyzed on 4 to 12% linear gradient precasted gel. The SDS-PAGE is transferred to a polyvinylidene fluoride membrane. The membrane is stained with Coomassie blue R-250. 20 kDa protein bands, corresponding to the correct size of human endostatin, are excised from a membrane blot and subjected to N-terminal protein sequencing analysis. The protein sequence of three major secreted proteins is determined, with 50% containing the amino acid sequence of human endostatin with the additional amino acid residues APQQEALA (SEQ ID NO: 5), 25% containing residues LA, and 25% containing no residues from human BM40 basement protein signal peptide. The 20 kDa protein is not found in the supernatant protein from Av3NuII cells. The results demonstrate that S8 cells transduced with Av3bmhendlx express and secrete human endostatin after it is processed from human BM40 basement protein signal peptide.

### EXAMPLE 7: INHIBITION OF RETINAL LEAKAGE, THICKENING, DETACHMENT, AND NEOVASCULARIZATION IN VIVO By BIV VECTOR MEDIATED ANTI-ANGIOGENESIS GENE EXPRESSION

Bovine immunodeficiency viral (BIV) vectors encoding eGFP are generated from the three component system (described in published international patent application number W00144458, the disclosure of which is incorporated by reference herein in its entirety). The transfer vector BIVendostatin was derived from pBSV4MGpptGAG, a BIV-based transfer vector construct encoding eGFP under MND U3 promoter according to Takahashi, K. et al. Hum. Gene Ther. 13, 1305-1316 (2002).. The eGFP coding sequence was replaced with murine endostatin (mEndo). Specifically, to delete eGFP from the parent plasmid pBSV4MGpptGAG, the eGFP plus some flanking sequence was amplified by PCR. The primers used were: eGFP1FOR 5'-GCGCATGTCGACAGAATATGGGCCAAAC-3', which incorporated a SalI site to the 5' end of the PCR product, and eGFP1 REV 5'-GCGCTACTGCAGAGCTAATGAGCTACAC-3', which incorporated a PstI site to the 3' end of the PCR product. This fragment was cut with SalI and PstI and ligated with pBSII(KS+) which was also previously digested with SalI and PstI, creating pBS2eGFP. The ExSite PCR-Based Site-Directed Mutagenesis Kit (Stratagene, LaJolla, CA) was used to delete eGFP from pBS2eGFP. PCR primers were designed that flanked the outer portions of the eGFP gene, and pointed outward, thus amplifying everything, including the entire flanking sequence and plasmid, except eGFP. The primers used were: DELeGFP1FOR: 5'-CCGGCTAGCTTAAGGGTGGCGACCGGT-3', which added NheI and AfIII restriction sites, and DELeGFP1REV: 5'-GCTTCGAACGCGTAGCGGCCAACCCTC-3', which added BstBI and Mlul restriction sites. The amplicon was treated according to the manufacturer's instruction, and ligated to form pBS2deleGFP with eGFP deleted but with the flanking sequence from the parental plasmid remaining. This strategy also created four new restriction sites in the middle. This fragment was sequenced to ensure no mutations had been introduced. The mEndo gene insert was prepared from an adenoviral shuttle plasmid, pAVmEndolxr according to Mori, K. et al. Amer. J. Pathol. 159, 313-320 (2001).. This plasmid was digested with NheI and Clal to release the mEndo fragment. This fragment was ligated with pBS2deleGFP, which was previously digested with NheI and BstBI (compatible ends with ClaI), generating pBS2deleGFPmEndo. The Woodchuck Hepatitis Virus post-transcriptional regulatory element (WPRE) was inserted downstream of mEndo at the Mlul site, creating pBS2deleGFPmEndoPRE. Finally, the plasmid pBS2deleGFPmEndoPRE was digested with Bsu36I and BbvCI to release the mEndo coding sequence and the orignal eGFP flanking sequences, and this fragment was ligated with pBS4MGpptGAG which was previously digested with Bsu36I and BbvCI, generating pBvMNDmEndoPRE. A null BIV vector, pBvMNDPRE, was also generated and served as a negative control vector. PBvMNDPRE was identical to pBvMNDmEndPRE except for the absence of the mEndo coding sequence.
Briefly, to generate BIV vector particles encoding mEndo (BIVendostatin), 293T cells in 150 mm dish (2 x 10⁷ cells/dish) were transfected with 45 µg pBvMNDmEndoPRE, 45 µg BIV-based packaging construct pBIVminipack, and 13.5 µg pseudotyping envelope expression construct pVSV-G. To generate BIV null vectors (BIVNuII), pBvMNDmEndoPRE was replaced with pBvMNDPRE construct. Forty eight hours post-transfection, the vector was harvested and filtered through a 0.45 µm filter. The vector supernatant was then concentrated by ultracentrifugation. The concentrated vector was aliquoted and stored at -80°C until used.

The concentrated vector was assayed for reverse transcriptase (RT) activity, a measure of vector particles. Both BIVendostatin and BIVNuII vectors scored RT activity of approximately 15 µg per ml. We have previously shown with a BIV-based vector encoding eGFP, that one ng of RT equals approximately 1x10⁵ transducing units (T.U.). Therefore, the estimated titer of BIVendostatin vector was 1.5 X 10⁹ T.U./ml. To examine if the BIVendostatin vector could mediate efficient production of mEndo, Cf2Th cells in a 6-well plate (4X10⁵ cells/well) were transduced with either 1 µl (15 ng RT equivalent vector particles) of BIVNuII vector or the same amount of BIVendostatin vector. Forty eight hours post-transduction, the cell supernatant was assayed for endostatin expression using a commercial endostatin assay kit, Accucyte Mouse Endostatin assay system, following the manufacturer's instruction (Cytimmune Sciences Inc., College Park, MD). BIVendostatin vector-transduced cells produced 412 ng/ml of endostatin while the BIVNuII vector did not score detectable level of endostatin.

A BIV vector encoding murine endostatin prepared according to O'Reilly et al., Cell;88(2):277-85 (1997) is administered via subretinal injection of singly transgenic mice (IRBP/rtTA-TRE/VEGF tgMICE) that express Vascular Endothelial Growth Factor from mouse photoreceptor cells upon induction with Doxycyclin. BIV vectors are injected into mouse right eyes while the left eyes serve as controls without injection of vectors. Three weeks after vector injection. 0.5 mg/ml of Doxycyclin is placed in the drinking water for the transgenic mice. It is found that Doxycyclin-induced VEGF expression results in severe neovascularization on the left eyes of the transgenic mice by examination of fluorescein angiograms. VEGF-induced neovascularization is completely blocked by BIV vector-mediated endostatin expression in the right eyes in the same animals.
Immunohistochemically stained ocular frozen sections from the eyes of adult C57BU6 mice given subretinal injections of 1.5 x10⁶ transducing units (TU) of BIVendostatin in the right eye and 1.5 x 10⁶ TU of BIVNuII in the left eye, euthanized four weeks after vector injection showed that eyes injected with BIVendostatin show heavy staining for endostatin in RPE cells and throughout the inner nuclear layer with dense staining of the walls of some blood vessels. Linear stained structures in the inner plexiform layer are typical of Muller cell processes. Eyes injected with BIVNuII showed reaction product along the internal limiting membrane and Bruch's membrane which is likely to be due to cross-reactivity with collagen XVIII.
Adult double transgenic rho/rtTA-TRE/VEGF mice given a subretinal injection of 1.5 x 10⁶ transducing units (TU) of BIVendostatin in the right eye and 1.5 x 10⁶ TU of BIVNuII in the left eye and started on 2 mg/ml of doxycycline in their drinking water four weeks after vector injection were assayed for retinal vascular permeability was measured using [3H]mannitol after 3 days on doxycycline. The retina to lung (RLLR) is significantly reduced in eyes expressing endostatin compared to fellow eyes that are injected with null vector, indicating that expression of endostatin with either of 2 different vector systems results in suppression of VEGF-induced vascular permeability in the retina.

Adult rho/rtTA-TRE/VEGF mice, given subretinal injections of 1.5 x 10⁶ TU of BIVendostatin in the right eye and 1.5 x 10⁶ TU of BIVNuII in the left eye and started on 0.5 mg/ml of doxycycline in their drinking water four weeks after vector injection are subjected to fluorescein angiography four days after initiation of doxycycline. Mice injected with BIVendostatin show normal appearing retinal vessels with distinct walls indicating little or no permeation of fluorescein in BIVendostatin-injected eyes, while BIVNuII-injected eyes show diffuse fluorescence throughout the retina indicating extensive extravasation of fluorescein. Seven days after initiation of doxycycline, there is still little evidence of fluorescein leakage in BIVendostatin-injected eyes, compared to massive leakage in BIVNuII-injected eyes.
When retinal vascular permeability is increased, fluid collects in the retina resulting in retinal thickening. Thickening in the macula is called macular edema and there is an inverse correlation between amount of abnormal thickening and visual acuity. Thus, retinal thickening is a physiologically relevant variable to assess and lends itself to precise quantitation using in vivo imaging techniques such as optical coherence tomography (OCT) or retinal thickness analysis (RTA). Seven days after starting 0.5 mg/ml of doxycycline in the drinking water to induce VEGF expression, frozen sections of BIVendostatin-injected eyes show less retinal thickening than BIVNuII-injected eyes. Eight rho/rtTA-TRE/VEGF mice are used to quantitatively assess the effect of endostatin on VEGF-induced retinal thickness. Four weeks after subretinal injection of 1.5 x 10⁶ TU of BIVendostatin in the right eye and 1.5 x10⁶ TU of BIVNuII in the left eye, mice are started on 0.5 mg/ml of doxycycline in their drinking water. Seven days after starting doxycycline, retinal thickness is measured by image analysis 300 µm from each edge of the optic nerve along the horizontal meridian and averaged to give a single experimental value in each eye. Mean retinal thickness is significantly greater in BIVNuII-injected eyesthan in BIVendostatin-injected eyes.

Four weeks after subretinal injection of 1.5 x 10⁶ TU of BIVendostatin in the right eye and 1.5 x 10⁶ TU of BIVNuII in the left eye, mice are started on 0.5 mg/ml of doxycycline in their drinking water. Seven days after starting doxycycline, the amount of retinal neovascularization is significantly greater in BIVNuII-injected eyes compared to BIVendostatin-injected eyes.

When rho/rtTA-TRE/VEGF mice are given 2 mg/ml of doxycycline in their drinking water for 5 days or longer, they express high levels of VEGF and develop severe neovascularization and retinal detachment. Eleven rho/rtTA-TRE/VEGF mice are used to assess the effect of BIV-vectored endostatin on this severe phenotype resulting from high levels of retinal VEGF. Four weeks after subretinal injection of 1.5 x10⁶ TU of BIVendostatin in the right eye and 1.5 x 10⁶ TU of BIVNuII in the left eye, mice are started on 2 mg/ml of doxycycline in their drinking water. Seven days after starting doxycycline, mice are euthanized and eyes were fixed in 2% paraformaldehyde. After gross pathologic examination, they are frozen in OCT and sectioned. Serial sections are stained with hematoxylin and eosin. Observers masked with respect to vector group determine whether each eye had a total, partial, or no retinal detachment. There are significantly fewer total retinal detachments in eyes injected with BIVendostatin than eyes injected with BIVNull. Four BIVendostatin-injected eyes have no retinal detachment compared to only one BIVNull-injected eye.

### EXAMPLE 8: INTRAOCULAR INJECTIONS

For studies using the adenoviral vectors, adult mice were given a subretinal injection of 6 x10⁷ particles of a 1:1 mixture of AGVC7mEndo and AGVas521 in one eye and 6 x 10⁷ particles of AGVnull in the other eye. For studies using the lentiviral vectors, the mice received 5x10⁶ transducing units (TU) of BIVendostatin in one eye and 5 x 10⁶ TU of BIVNull in the other eye. Pulled glass micropipets were calibrated to deliver 1 µl of vehicle upon depression of a foot switch. Injections were performed using a condensing lens system on the dissecting microscope and a contact lens, which allowed visualization of the retina during the injection. The mice were anesthetized, pupils were dilated, and under a dissecting microscope, the sharpened tip of the micropipet was passed through the sclera posterior to the limbus and was positioned just above the retina. Depression of the foot switch caused the jet of injection fluid to penetrate the retina. This technique is very atraumatic and the direct visualization allows confirmation that the injection was successful, because of the appearance of a small retinal detachment (bleb). The blebs were quite uniform in size and involved slightly less than half of the retina.

### EXAMPLE 9: GUTLESS ADENOVIRAL VECTOR-MEDIATED REGULATED ENDOSTATIN EXPRESSION IN THE EYE

Regulated expression of endostatin in vivo using an adenoviral vector delivery system is achieved according to methods described by Xu et al., Molecular Therapy; 3:262 (2001). The AGV vectors encoding the tamoxifen-inducible chimeric transcription factor, AGVas521, and the regulatable endostatin transgene, AGVC7mEndo, were generated from the plasmids, pAGVas521 and pAGVC7mEndo, respectively. In addition to the transgene expression cassette (see below), the AGV plasmids contain the left and right ITRs flanked by unique Pac I sites, the packaging signal of Ad5, and approximately 25 kb of the human alpha synuclein intronic region as a DNA "stuffer" according to Reddy, P.S. et al., Molec. Ther. 5, 63-73 (2002). The plasmid pAGVas521, contains the tamoxifen-inducible chimeric transcription factor composed of the unique zinc finger DNA binding domain, a modified ligand binding domain based on the human estrogen receptor, and the transactivating region derived from VP16 driven by the CMV promoter. To construct pAGVas521, the chimeric transcription factor expression cassette was isolated from pAvCv-C7LBD by digestion with Nru I and Bam HI, and inserted into pBLSV2. The plasmid pBLSV2 was derived from pBluescript (Strategene, La Jolla, CA), and contains two multicloning site polylinkers. The resulting plasmid, pBLSV2as521 was digested with Bspe I and ligated to pGTI.24aPL2 digested with Xma I, to generated pGTI24as521. pGTI24aPL2 contains a multicloning site polylinker flanked by human synuclein stuffer DNA. Next, pGTI24as521 was digested with Pac I to liberate the plasmid backbone, and combined with Pme I-Mlu I digested pBV2, usinghomologous recombination in BJ5138 E. coli according to Toietta, G. et al., Mol. Ther. 5, 204-210 (2002), to generate pAGVas521. The plasmid pBV2 contains 26625 bp of human synuclein stuffer DNA.

The plasmid pAGVC7mEndo contains the ligand-inducible transcription factor regulated promoter driving the expression of murine endostatin. To construct pAGVC7mEndo, the plasmid pav-6X2C7tatamendo was digested with Asc I (blunted) and Bam HI and inserted into pBLSV2C7endo. The plasmid pBLSV2C7endo was digested with Bam HI and Eco RI, ends filled in, and ligated to pGTI245.aPL2 digested with Sma I to generated pGTI24C7endo. Finally, pGTI245.aPL2 was digested with Pac I to liberate the plasmid backbone, and combined with Pme I and Mlu I digested pBV4, using homologous recombination in BJ5138 E. coli per Toietta, supra, to generate pAGVC7mEndo. The plasmid pBV4 contains 27191 bp of human synuclein stuffer DNA.

Gutless vector generation and large scale production and purification were performed as described by Reddy et al. The particle titers were determined by optical density measurements. DNA extracted from CsCI-purified vectors were analyzed by restriction enzyme digestions to verify vector integrity. A hexon-based quantitative PCR assay was used to determine the level of helper virus contamination in AGV preparations. Helper contamination levels of AGVNull, AGVas521, and AGVC7mEndo preparations used in this study were 0.09%, 1.9%, and 1.4%, respectively.

This tamoxifen-inducible system displays high-level inducible endostatin expression in the serum of C57BU6 mice following systemic administration of early generation, E1/E2a/E3-deficient vectors encoding either the inducible transcription factor, or the regulatable endostatin transgene.

This regulatory system is composed of two components, an inducible transcription factor, and a responsive promoter driving expression of mouse endostatin. The transcription factor consists of a modified human estrogen ligand binding domain that is responsive to tamoxifen, a unique cysteine 2-histidine 2 zinc finger DNA binding motif, and a minimal transactivation domain from VP 16. The responsive promoter consists of 6 repeats of the DNA sequence recognized by the transcription factor DNA binding domain (DBD) and a DNA encoding endostatin. In the presence of tamoxifen, this transcription factor activates transcription from a unique target nucleic acid sequence linked to a minimal promoter. When evaluated with a luciferase reporter in vitro, tamoxifen induces expression up to 250 fold. This system is incorporated into two gutless adenoviral vectors, which are devoid of all viral coding regions. One vector encodes the transcription factor, and the second encodes the target promoter driving transcription of a nucleic acid encoding endostatin. The two vectors are injected into mice, which results in efficient liver transduction. Administration of tamoxifen to the mice results in inducible expression of endostatin, yielding extremely high plasma levels of up to 20 µg/ml. Tamoxifen induction is achieved four times over a two-month period. In the absence of tamoxifen, background levels of endostatin are observed.

Mice injected with the vector pair of AGVC7mEndo and AGVas521 constituting the inducible system treated with tamoxifen showed prominent staining for endostatin throughout the entire retina, demonstrating strong induction of endostatin expression in the retina.

Retinal vascular permeability was measured using [3H]mannitol as tracer in adult IRBP/rtTA-TRE/VEGF mice given a subretinal injection of 6 x 10⁷ particles of a 1:1 mixture of AGVC7mEndo and AGVas521 in the right eye and 6x10⁷ particles of AGVNull in the left eye, followed by treatment with tamoxifen for six days to induce endostatin expression, the last 3 of which the mice also received doxycycline to induce VEGF expression. Both the retina to lung (RLLR) and retina to renal leakage ratios (RRLR) are significantly reduced in eyes with induced expression of both endostatin and VEGF compared to fellow eyes that had induced expression of VEGF alone. This demonstrates that endostatin suppresses VEGF-induced increased permeability of retinal vessels.

### EXAMPLE 10: TRANSGENIC MICE AND ASSAY METHODS

Two lines of double transgenic mice with inducible expression of VEGF in the retina have been generated by Ohno-Matsui, K. et al. Am. J. Pathol. 160, 711-719 (2002) were used in this study. In one of the lines, the interphotoreceptor retinoid binding protein (IRBP) promoter is combined with the reverse tetracycline transactivator (rtTA) system to direct doxycyline-inducible expression of VEGF in photoreceptors. These are referred to as IRBP/rtTA-TRE/VEGF mice. In the second line of double transgenic mice, the rhodopsin promoter rather than the IRBP promoter is combined with the reverse tetracycline transactivator system to direct doxycyline-inducible expression of VEGF in photoreceptors. These are referred to as rho/rtTA-TRE/VEGF mice.

### 1) Immunohistochemistry for endostatin

Eyes were punctured and placed in 4% paraformaldehyde/5% sucrose and then incubated overnight in 0.1 M phosphate buffer, pH 7.4 for 1.5 hours at 4°C. Eyes were then rinsed and rapidly frozen in a 2:1 mixture of 0.1 M phosphate buffer/20 % sucrose in OCT. Ten µm frozen sections were dried and post-fixed in cold 4 % paraformaldehyde for 30 minutes. After rinsing, slides were blocked with cold methanol containing 6.25% H2O2 for 15 minutes and then with 2 % skim milk in Tris-buffered saline (TBS) for 30 minutes at room temperature. Slides were incubated in 1.5 µg/ml of polyclonal goat IgG directed against mouse endostatin (R&D Systems, Minneapolis, MN) in 2% milk/TBS for 1 hour at room temperature. After washing in 0.1 % milk/TBS for 10 minutes, slides were incubated 30 minutes at room temperature in 2 µg/ml biotin-conjugated anti-goat IgG (Santa Cruz Biotechnology, Santa Cruz, CA) in 2% milk/TBS. After washing 10 minutes in 0.1% milk/TBS, slides were incubated for 30 minutes at room temperature in streptavidin-phosphatase (Kirkegaard and Perry, Cabin John, MD). After 3 five minute washes in 0.05 M Tris HCl, slides were developed with HistoMark Red (Kirkegaard and Perry) and mounted.

### 2) Measurement of retinal vascular permeability using [³H]mannitol as tracer

Adult double transgenic IRBP/rtTA-TRE/VEGF mice (n = 11) were given a subretinal injection of 6 x10⁷ particles of a 1:1 mixture of AGVC7mEndo and AGVas521 in the right eye and 6 x 10⁷ particles of AGVNull in the left eye. The next day mice were started on daily intraperitoneal injections of 500 µg of tamoxifen in 5% DMSO in sunflower oil and after 3 days, they were given 2 mg/ml of doxycycline in their drinking water. Three days after initiating doxycycline, retinal vascular permeability was measured using [³H]mannitol. Briefly, mice were given an intraperitoneal injection of 1 µCi / gram body weight of [3H]mannitol (New England Nuclear, Boston, MA). After one hour, mice were sacrificed and eyes were removed. The cornea and lens were removed and the entire retina was carefully dissected from the eyecup and placed within pre-weighed scintillation vials. The thoracic cavity was opened and the left superior lobe of the lung was removed and placed in another pre-weighed scintillation vial. A left dorsal incision was made and the retroperitoneal space was entered without entering the peritoneal cavity. The renal vessels were clamped with a forceps and the left kidney was removed, cleaned of all fat, and placed into a pre-weighed scintillation vial. All liquid was removed from the vials and remaining droplets were allowed to evaporate over 20 minutes. The vials were weighed and the tissue weights were recorded. One ml of NCSII solubilizing solution (Amersham, Chicago, IL) was added to each vial and the vials were incubated overnight in a 50°C water bath. The solubilized tissue was brought to room temperature and decolorized with 20% benzoyl peroxide in toluene in a 50°C water bath. The vials were brought to room temperature and 5 ml of Cytoscint ES (ICN, Aurora, OH) and 30 µl of glacial acetic acid were added. The vials were stored for several hours in darkness at 4°C to eliminate chemoluminescence. Radioactivity was counted with a Wallac 1409 Liquid Scintillation Counter (Gaithersburg, MD).

### 3) Assessment of retinal vascular permeability using fluorescein leakage

Adult rho/rtTA-TRE/VEGF double transgenic mice were given subretinal injections of 1.5 x 10⁶ transducing units (TU) of BIVendostatin in the right eye and 1.5 x10⁶ TU of BIVNull in the left eye. Four weeks after vector injection, mice were started on 0.5 mg/ml of doxycycline in their drinking water. Four or 7 days later, mice were given an intraperitoneal injection of 12 µl/g body weight of 1 % fluorescein sodium (Alcon, Fort Worth, Texas) and after 1 minute pictures were taken of each eye using in vivo fluorescence microscopy. Seven days after initiating VEGF expression, another mouse was euthanized and retinal frozen sections were cut through the posterior part of the retina adjacent to the optic nerve in the same location in each eye. The sections were stained with Griffonia simplicifolia lectin, hematoxylin, and eosin. The retina in the BIVNull injected eye (F and H) is much thicker than the retina in the BIVendostatin-injected eye (E and G).

### 4) Measurement of retinal thickness

Adult rho/rtTA-TRE/VEGF double transgenic mice were given subretinal injections of 1.5 x 10⁶ TU of BIVendostatin in the right eye and 1.5 x 10⁶ TU of BIVNull in the left eye. Four weeks after vector injection, mice were started on 0.5 mg/ml of doxycycline in their drinking water. Seven days after initiating doxycycline, mice were euthanized and 10 µm retinal frozen sections were cut through the posterior part of the retina adjacent to the optic nerve in the same location in each eye. The sections were stained with biotinylated Griffonia simplicifolia lectin B4 (GSA, Vector Laboratories, Burlingame, CA), which selectively binds to vascular cells 22. Slides were incubated in methanol/H2O2 for 10 minutes at 4°C, washed with 0.05 M Tris-buffered saline, pH 7.6 (TBS), and incubated for 30 minutes in 10% normal porcine serum. Slides were incubated 2 hours at room temperature with biotinylated GSA and after rinsing with 0.05M TBS, they were incubated with avidin coupled to peroxidase (Vector Laboratories) for 45 minutes at room temperature. The slides were developed with HistoMark Red (Kirkegaard and Perry) to give a red reaction product and counter stained with hematoxyln and eosin. Retinal thickness was measured by image analysis 300 µm from each edge of the optic nerve and averaged to give a single experimental value.

### 5) Assessment of retinal neovascularization in double transgenic mice

Three rho/rtTA-TRE/VEGF mice were used to assess the effect of endostatin on VEGF-induced retinal neovascularization. Four weeks after subretinal injection of 1.5 x 10⁶ TU of BIVendostatin in the right eye and 1.5 x 10⁶ TU of BIVNull in the left eye, mice were started on 0.5 mg/ml of doxycycline in their drinking water. Seven days after starting doxycycline, mice were euthanized and each eye was sectioned (10 µm sections) from the peripheral edge of the iris to the other peripheral edge 180° away. Sections were stained with Griffonia simplicifolia lectin (GSA), hematoxylin, and eosin.
The area of GSA staining in the photoreceptor layer is an indication of the amount of neovascularization and was measured on sections 100 µm apart from one edge of the iris to the other edge (generally 13 sections per eye). The average of these 13 measurements constituted a single experimental value, the area of neovascularization per section.

### 6) Assessment of retinal detachment in double transgenic mice

Eleven adult rho/rtTA-TRE/VEGF double transgenic mice were given subretinal injections of 1.5 x10⁶ TU of BIVendostatin in the right eye and 1.5 x 10⁶ TU of BIVNull in the left eye. Four weeks after vector injection, mice were started on 2 mg/ml of doxycycline in their drinking water. After 4 days, mice were anesthetized, pupils were dilated and funduscopic examination was performed on each eye, by two independent observers noting if there was a total or partial retinal detachment, or no detachment.

## Claims

1. An endostatin for use in a method of treatment of retinal edema or increased retinal vascular permeability in an individual afflicted therewith.

2. An endostatin for use according to claim 1, wherein said endostatin is a polypeptide with the amino acid sequence set forth in SEQ ID NO:1.

3. An endostatin for use according to claim 1, wherein the endostatin is a polypeptide fragment of the polypeptide with the amino acid sequence set forth in SEQ ID NO:1, wherein the fragment remains effective to treat retinal edema or wherein the endostatin is an endostatin analogue with at least 70% homology to the endostatin of SEQ ID NO:1.

4. An endostatin for use according to claim 1, wherein an increase in the amount of an endostatin in ocular tissues is effected.

5. An endostatin for use according to claim 4, wherein said increase is effected by administering an effective amount of a viral vector comprising an endostatin-encoding nucleic acid.

6. An endostatin for use according to claim 5, wherein said viral vector is selected from the group consisting of an adenovirus, an adeno-associated virus, a retrovirus, and a lentlvlrus.

7. An endostatin for use according to claim 1, comprising at least one microcapsule, wherein the microcapsule comprises cells that secrete endostatin and wherein said increase is effected by implanting said microcapsule within the individual being afflicted with retinal edema or increased retinal vascular permeability.

8. An endostatin for use according to claim 7, wherein the microcapsule comprises an alginate salt, for example sodium alginate or calcium alginate.

9. An endostatin for use according to claim 7, wherein the microcapsule comprises poly L-lysine.

10. An endostatin for use according to claim 7, wherein the cells comprise an exogenous endostatin-encoding nucleic acid.

11. An endostatin for use according to claim 7, wherein the cells overexpress an endogenous endostatin-encoding gene.

12. An endostatin for use according to claim 1, which is administered at between about 2.5 mg/kg per day and about 20 mg/kg per day of endostatin.

13. An endostatin for use according to claim 6 wherein the vector is an adenoviral vector or a lentiviral vector and is administered in an amount effective to provide for expression of endostatin by the individual to result in a concentration of endostatin of up to 1,000,000 ng/ml in the serum of the individual and preferably of at least about 300 ng/ml in the serum of the individual.

14. An endostatin for use according to claim 7 wherein the microcapsules are implanted in an amount effective to provide for expression of endostatin by the individual to result in a concentration of endostatin of up to 1,000,000 ng/ml in the serum of the individual and preferably of at least about 300 ng/ml in the serum of the individual"

15. An endostatin for use according to claim 13 or 14 wherein endostatin is expressed in the individual in a sufficient amount to result in a concentration of endostatin of about 300 ng/ml to about 3000 ng/ml in the serum of the individual and preferably in a concentration of endostatin of about 300 ng/ml to about 1500 ng/ml in the serum of the individual.

16. An endostatin for use according to claim 5, wherein the vector is administered in an amount of from about 10⁸ plaque forming units to about 10¹⁴ plaque forming units.

17. An endostatin for use according to claim 5, wherein the endostatin-encoding nucleic acid has the sequence set forth In SEQ ID NO:2.

18. An endostatin for use according to claim 5, wherein the endostatin-encoding nucleic acid encodes a polypeptide with the amino acid sequence of SEQ ID NO:1.

19. An endostatin for use according to claim 5, wherein the endoatatin-encoding nucleic acid encodes a polypeptide fragment of the polypeptide with the amino acid sequence set forth In SEQ ID NO:1, which fragment remains effective to treat retinal edema or wherein the endostatin is an endostatin analogue with at least 70% homology to the endostatin of SEQ ID NO:1.

20. An endostatin for use according to claim 6 wherein the vector is a lentiviral vector.

21. An endostatin for use according to claim 20 wherein the lentiviral vector is a bovine immunodeficiency viral vector.

22. An endostatin for use according to claim 5 or claim 20, wherein the viral vector is administered intraocularly, subretinally, intravitreally, or perioocularly.

23. An endostatin for use according to claim 5 wherein the vector is an adenoviral vector which comprises an inducible promoter operably linked to the endostatin coding region.

## Patentansprüche

1. Endostatin zur Verwendung in einem Verfahren zur Behandlung von Retinaödem oder erhöhter Retinagefäßpermeabilität bei einem daran leidenden Individuum.

2. Endostatin zur Verwendung nach Anspruch 1, worin das Endostatin ein Polypeptid mit der in Seq.-ID Nr. 1 dargelegten Aminosäuresequenz ist.

3. Endostatin zur Verwendung nach Anspruch 1, worin das Endostatin ein Polypeptidfragment des Polypeptids mit der in Seq.-ID Nr. 1 dargelegten Aminosäuresequenz ist, worin das Fragment weiterhin zum Behandeln von Retinaödem wirksam ist oder worin das Endostatin ein Endostatinanalogon mit zumindest 70 % Homologie zum Endostatin der Seq.-ID Nr. 1 ist.

4. Endostatin zur Verwendung nach Anspruch 1, worin eine Erhöhung der Menge eines Endostatins in okularen Geweben bewirkt wird.

5. Endostatin zur Verwendung nach Anspruch 4, worin die Erhöhung durch das Verabreichen einer wirksamen Menge eines Virusvektors, der eine für Endostatin kodierende Nucleinsäure umfasst, bewirkt wird.

6. Endostatin zur Verwendung nach Anspruch 5, worin der Virusvektor aus der aus einem Adenovirus, einem adeno-assoziierten Virus, einem Retrovirus und einem Lentivirus bestehenden Gruppe ausgewählt ist.

7. Endostatin zur Verwendung nach Anspruch 1, umfassend zumindest eine Mikrokapsel, worin die Mikrokapsel Zellen umfasst, die Endostatin sekretieren, und worin die Erhöhung durch das Implantieren der Mikrokapsel in das an Retinaödem oder erhöhter Retinagefäßpermeabilität leidende Individuum bewirkt wird.

8. Endostatin zur Verwendung nach Anspruch 7, worin die Mikrokapsel ein Alginat, z.B. Natriumalginat oder Calciumalginat, umfasst.

9. Endostatin zur Verwendung nach Anspruch 7, worin die Mikrokapsel Poly-L-lysin umfasst.

10. Endostatin zur Verwendung nach Anspruch 7, worin die Zellen eine exogene, für Endostatin kodierende Nucleinsäure umfassen.

11. Endostatin zur Verwendung nach Anspruch 7, worin die Zellen ein endogenes, für Endostatin kodierendes Gen überexprimieren.

12. Endostatin zur Verwendung nach Anspruch 1, welches zu zwischen etwa 2,5 mg/kg pro Tag und etwa 20 mg/kg Endostatin pro Tag verabreicht wird.

13. Endostatin zur Verwendung nach Anspruch 6, worin der Vektor ein Adenovirusvektor oder ein Lentivirusvektor ist und in einer Menge verabreicht wird, die dahingehend wirksam ist, die Expression von Endostatin durch das Individuum zu ermöglichen, was zu einer Endostatinkonzentration von bis zu 1.000.000 ng/ml im Serum des Individuums und vorzugsweise von mindestens etwa 300 ng/ml im Serum des Individuums führt.

14. Endostatin zur Verwendung nach Anspruch 7, worin die Mikrokapseln in einer Menge implantiert werden, die dahingehend wirksam ist, die Expression von Endostatin durch das Individuum zu ermöglichen, was zu einer Endostatinkonzentration von bis zu 1.000.000 ng/ml im Serum des Individuums und vorzugsweise von mindestens etwa 300 ng/ml im Serum des Individuums führt.

15. Endostatin zur Verwendung nach Anspruch 13 oder 14, worin Endostatin im Individuum in einer ausreichenden Menge exprimiert wird, um zu einer Endostatinkonzentration von etwa 300 ng/ml bis etwa 3.000 ng/ml im Serum des Individuums und vorzugsweise zu einer Endostatinkonzentration von etwa 300 ng/ml bis etwa 1.500 ng/ml im Serum des Individuums zu führen.

16. Endostatin zur Verwendung nach Anspruch 5, worin der Vektor in einer Menge von etwa 10⁸ infektiösen Einheiten bis etwa 10¹⁴ infektiösen Einheiten verabreicht wird.

17. Endostatin zur Verwendung nach Anspruch 5, worin die für Endostatin kodierende Nucleinsäure die in Seq.-ID Nr. 2 dargelegte Sequenz aufweist.

18. Endostatin zur Verwendung nach Anspruch 5, worin die für Endostatin kodierende Nucleinsäure für ein Polypeptid mit der Aminosäuresequenz von Seq.-ID Nr. 1 kodiert.

19. Endostatin zur Verwendung nach Anspruch 5, worin die für Endostatin kodierende Nucleinsäure für ein Polypeptidfragment des Polypeptids mit der in Seq.-ID Nr. 1 dargelegten Aminosäuresequenz kodiert, welches Fragment weiterhin dahingehend wirksam ist, Retinaödem zu behandeln, oder worin das Endostatin ein Endostatinanalogon mit zumindest 70 % Homologie zum Endostatin der Seq.-ID Nr. 1 ist.

20. Endostatin zur Verwendung nach Anspruch 6, worin der Vektor ein Lentivirusvektor ist.

21. Endostatin zur Verwendung nach Anspruch 20, worin der Lentivirusvektor ein Vektor des bovinen Immunschwächevirus ist.

22. Endostatin zur Verwendung nach Anspruch 5 oder Anspruch 20, worin der Virusvektor intraokular, subretinal, intravitreal oder periokular verabreicht wird.

23. Endostatin zur Verwendung nach Anspruch 5, worin der Vektor ein Adenovirusvektor ist, der einen induzierbaren Promotor umfasst, der mit der für Endostatin kodierenden Region operabel verbunden ist.

## Revendications

1. Endostatine pour utilisation dans une méthode de traitement d'un oedème rétinien ou d'une perméabilité vasculaire rétinienne accrue dans un individu qui en est affecté.

2. Endostatine pour utilisation selon la revendication 1, où ladite endostatine est un polypeptide avec la séquence des acides aminés telle qu'exposée dans SEQ ID NO:1.

3. Endostatine pour utilisation selon la revendication 1, où l'endostatine est un fragment du polypeptide avec la séquence des acides aminés exposée dans SEQ ID NO:1, où le fragment reste efficace pour traiter l'oedème rétinien, ou bien où l'endostatine est un analogue d'endostatine avec au moins 70% d'homologie à l'endostatine de la SEQ ID NO:1.

4. Endostatine pour utilisation selon la revendication 1, où une augmentation de la quantité d'une endostatine dans les tissus occulaires est effectuée.

5. Endostatine pour utilisation selon la revendication 4, où ladite augmentation est effectuée en administrant une quantité efficace d'un vecteur viral comprenant un acide nucléique codant pour l'endostatine.

6. Endostatine pour utilisation selon la revendication 5, où ledit vecteur viral est sélectionné dans le groupe consistant en un adénovirus, un virus adéno-associé, un rétrovirus et un lentivirus.

7. Endostatine pour utilisation selon la revendication 1, comprenant au moins une microcapsule, où la microcapsule comprend des cellules qui sécrètent de l'endostatine, et où ladite augmentation est effectuée en implantant ladite microcapsule dans l'individu affecté d'oedème rétinien ou d'une perméabilité vasculaire rétinienne accrue.

8. Endostatine pour utilisation selon la revendication 7, où la microcapsule comprend un sel d'alginate, par exemple un alginate de sodium ou alginate de calcium.

9. Endostatine pour utilisation selon la revendication 7, où la microcapsule comprend poly L-lysine.

10. Endostatine pour utilisation selon la revendication 7, où les cellules comprennent un acide nucléique exogène codant pour l'endostatine.

11. Endostatine pour utilisation selon la revendication 7, où les cellules surexpriment un gène endogène de codage d'endostatine.

12. Endostatine pour utilisation selon la revendication 1, qui est administrée entre environ 2,5 mg/kg par jour et environ 20 mg/kg par jour d'endostatine.

13. Endostatine pour utilisation selon la revendication 6, où le vecteur est un vecteur adénoviral ou un vecteur lentiviral et est administré en une quantité efficace pour réaliser l'expression de l'endostatine par l'individu pour obtenir une concentration d'endostatine jusqu'à 1 000 000 ng/ml dans le sérum de l'individu et de préférence au moins environ 300 ng/ml dans le sérum de l'individu.

14. Endostatine pour utilisation selon la revendication 7, où les microcapsules sont implantées en une quantité efficace pour , réaliser l'expression de l'endostatine par l'individu pour obtenir une concentration d'endostatine jusqu'à 1 000 000 ng/ml dans le sérum de l'individu et de préférence au moins environ 300 ng/ml dans le sérum de l'individu.

15. Endostatine pour utilisation selon la revendication 13 ou 14, où l'endostatine est exprimée dans l'individu en une quantité suffisante pour obtenir une concentration d'endostatine d'environ 300 ng/ml à environ 3000 ng/ml dans le sérum de l'individu et de préférence dans une concentration d'endostatine d'environ 300 ng/ml à environ 1500 ng/ml dans le sérum de l'individu.

16. Endostatine pour utilisation selon la revendication 5, où le vecteur est administré en une quantité d'environ 10⁸ unités formant des plages à environ 10¹⁴ unités formant des plages.

17. Endostatine pour utilisation selon la revendication 5, où l'acide nucléique codant pour l'endostatine a la séquence exposée dans SEQ ID NO:2.

18. Endostatine pour utilisation selon la revendication 5, où l'acide nucléique codant pour l'endostatine code pour un polypeptide avec la séquence des acides aminés de la SEQ ID NO:1.

19. Endostatine pour utilisation selon la revendication 5, où l'acide nucléique codant pour l'endostatine code un fragment du polypeptide avec la séquence des acides aminés exposée dans SEQ ID NO:1, ledit fragment reste efficace pour traiter un oedème rétinien, ou bien où l'endostatine est un analogue d'endostatine avec au moins 70% d'homologie avec l'endostatine de SEQ ID NO:1.

20. Endostatine pour utilisation selon la revendication 6, où le vecteur est un vecteur lentiviral.

21. Endostatine pour utilisation selon la revendication 20, où le vecteur lentiviral est un vecteur viral d'immunodéficience bovine.

22. Endostatine pour utilisation selon la revendication 5 ou la revendication 20, où le vecteur viral est administré de manière intraocculaire, sous-rétinienne, intravitréenne ou périoocculaire.

23. Endostatine pour utilisation selon la revendication 5, où le vecteur est un vecteur adénoviral qui comprend un promoteur inductible fonctionnellement lié à la région de codage de l'endostatine.


### SEQUENCE LISTING

<110> Novartis AG
<120> OCULAR GENE THERAPY
<130> 4-32625
   <160> 21
<170> FastSEQ for Windows Version 4.0
<210> 1
   <211> 183
   <212> PRT
   <213> Human
<400> 1
   His Ser His Arg Asp Phe Gln Pro Val Leu His Leu Val Ala Leu Asn
    1               5                  10                  15
   Ser Pro Leu Ser Gly Gly Met Arg Gly Ile Arg Gly Ala Asp Phe Gln
               20                  25                  30
   Cys Phe Gln Gln Ala Arg Ala Val Gly Leu Ala Gly Thr Phe Arg Ala
           35                  40                  45
   Phe Leu Ser Ser Arg Leu Gln Asp Leu Tyr Ser Ile Val Arg Arg Ala
       50                  55                  60
   Asp Arg Ala Ala Val Pro Ile Val Asn Leu Lys Asp Glu Leu Leu Phe
   65                  70                  75                  80
   Pro Ser Trp Glu Ala Leu Phe Ser Gly Ser Glu Gly Pro Leu Lys Pro
                   85                  90                  95
   Gly Ala Arg Ile Phe Ser Phe Asp Gly Lys Asp Val Leu Arg His Pro
               100                 105                 110
   Thr Trp Pro Gln Lys Ser Val Trp His Gly Ser Asp Pro Asn Gly Arg
           115                 120                 125
   Arg Leu Thr Glu Ser Tyr Cys Glu Thr Trp Arg Thr Glu Ala Pro Ser
       130                 135                 140
   Ala Thr Gly Gln Ala Ser Ser Leu Leu Gly Gly Arg Leu Leu Gly Gln
   145                 150                 155                 160
   Ser Ala Ala Ser Cys His His Ala Tyr Ile Val Leu Cys Ile Glu Asn
                   165                 170                 175
   Ser Phe Met Thr Ala Ser Lys
               180
<210> 2
   <211> 551
   <212> DNA
   <213> Human
<400> 2
   acagccaccg cgacttccag ccggtgctcc acctggttgc gctcaacagc cccctgtcag      60
   gcggcatgcg gggcatccgc ggggccgact tccagtgctt ccagcaggcg cgggccgtgg     120
   ggctggcggg caccttccgc gccttcctgt cctcgcgcct gcaggacctg tacagcatcg     180
   tgcgccgtgc cgaccgcgca gccgtgccca tcgtcaacct caaggacgag ctgctgtttc     240
   ccagctggga ggctctgttc tcaggctctg agggtccgct gaagcccggg gcacgcatct     300
   tctcctttga cggcaaggac gtcctgaggc accccacctg gccccagaag agcgtgtggc     360
   atggctcgga ccccaacggg cgcaggctga ccgagagcta ctgtgagacg tggcggacgg     420
   aggctccctc ggccacgggc caggcctcct cgctgctggg gggcaggctc ctggggcaga     480
   gtgccgcgag ctgccatcac gcctacatcg tgctctgcat tgagaacagc ttcatgactg     540
   cctccaagta g                                                          551
<210> 3
   <211> 207
   <212> PRT
   <213> Mouse
<400> 3
   Met Glu Thr Asp Thr Leu Leu Leu Trp Val Leu Leu Leu Trp Val Pro
   1               5                   10                  15
   Gly Ser Thr Gly Asp Ala Ala His Thr His Gln Asp Phe Gln Pro Val
               20                  25                  30
   Leu His Leu Val Ala Leu Asn Thr Pro Leu Ser Gly Gly Met Arg Gly
           35                  40                  45
   Ile Arg Gly Ala Asp Phe Gln Cys Phe Gln Gln Ala Arg Ala Val Gly
       50                  55                  60
   Leu Ser Gly Thr Phe Arg Ala Phe Leu Ser Ser Arg Leu Gln Asp Leu
   65                  70                  75                  80
   Tyr Ser Ile Val Arg Arg Ala Asp Arg Gly Ser Val Pro Ile Val Asn
                   85                  90                  95
   Leu Lys Asp Glu Val Leu Ser Pro Ser Trp Asp Ser Leu Phe Ser Gly
               100                 105                 110
   Ser Gln Gly Gln Leu Gln Pro Gly Ala Arg Ile Phe Ser Phe Asp Gly
           115                 120                 125
   Arg Asp Val Leu Arg His Pro Ala Trp Pro Gln Lys Ser Val Trp His
       130                 135                 140
   Gly Ser Asp Pro Ser Gly Arg Arg Leu Met Glu Ser Tyr Cys Glu Thr
   145                 150                 155                 160
   Trp Arg Thr Glu Thr Thr Gly Ala Thr Gly Gln Ala Ser Ser Leu Leu
                   165                 170                 175
   Ser Gly Arg Leu Leu Glu Gln Lys Ala Ala Ser Cys His Asn Ser Tyr
               180                 185                 190
   Ile Val Leu Cys Ile Glu Asn Ser Phe Met Thr Ser Phe Ser Lys
           195                 200                 205
<210> 4
   <211> 624
   <212> DNA
   <213> Mouse
<400> 4
   atggagacag acacactcct gctatgggta ctgctgctct gggttccagg ttccactggt     60
   gacgcggccc atactcatca ggactttcag ccagtgctcc acctggtggc actgaacacc    120
   cccctgtctg gaggcatgcg tggtatccgt ggagcagatt tccagtgctt ccagcaagcc    180
   cgagccgtgg ggctgtcggg caccttccgg gctttcctgt cctctaggct gcaggatctc    240
   tatagcatcg tgcgccgtgc tgaccggggg tctgtgccca tcgtcaacct gaaggacgag    300
   gtgctatctc ccagctggga ctccctgttt tctggctccc agggtcaagt gcaacccggg    360
   gcccgcatct ttctctttga cggcagagat gtcctgagac acccagcctg gccgcagaag    420
   agcgtatggc acggctcgga ccccagtggg cggaggctga tggagagtta ctgtgagaca    480
   tggcgaactg aaactactgg ggctacaggt caggcctcct ccctgctgtc aggcaggctc    540
   ctggaacaga aagctgcgag ctgccacaac agctacatcg tcctgtgcat tgagaatagc    600
   ttcatgacct ctttctccaa atag                                           624
<210> 5
   <211> 8
   <212> PRT
   <213> Human
<400> 5
   Ala Pro Gln Gln Glu Ala Leu Ala
   1 5
<210> 6
   <211> 38
   <212> DNA
   <213> Artificial Sequence
<220>
   <223> PCR Primer
<400> 6
   actggtgacg cggcccatac tcatcaggac tttcagcc 38
<210> 7
   <211> 32
   <212> DNA
   <213> Artificial Sequence
<220>
   <223> PCR Primer
<400> 7
   aagggctatc gatctagctg gcagaggcct at 32
<210> 8
   <211> 20
   <212> DNA
   <213> Artificial Sequence
<220>
   <223> PCR Primer
<400> 8
   cactgcttac tggcttatcg 20
<210> 9
   <211> 29
   <212> DNA
   <213> Artificial Sequence
<220>
   <223> PCR Primer
<400> 9
   ctgatgagta tgggccgcgt caccagtgg 29
<210> 10
   <211> 32
   <212> DNA
   <213> Artificial Sequence
<220>
   <223> PCR Primer
<400> 10
   aagggctatc gatctagctg gcagaggcct at 32
<210> 11
   <211> 35
   <212> DNA
   <213> Artificial Sequence
<220>
   <223> PCR Primer
<400> 11
   gatctctaga ccaccatgca tactcatcag gactt 35
<210> 12
   <211> 30
   <212> DNA
   <213> Artificial Sequence
<220>
   <223> PCR Primer
<400> 12
   actggagaaa gaggtttatc tagctactag 30
<210> 13
   <211> 18
   <212> PRT
   <213> Adenovirus
<400> 13
   Met Arg Tyr Met Ile Leu Gly Leu Leu Ala Leu Ala Ala Val Cys Ser
    1               5                   10                  15
   Ala Ala
<210> 14
   <211> 96
   <212> DNA
   <213> Artificial Sequence
<220>
   <223> PCR Primer
<400> 14
   gatctctaga ccaccatgag gtacatgatt ttaggcttgc tcgcccttgc ggcagtctgc    60
   agcgcggccc atactcatac tcatcaggac tttcag                              96
<210> 15
   <211> 29
   <212> DNA
   <213> Artificial Sequence
<220>
   <223> PCR Primer
<400> 15
   atcgatcata ctcatcagga ctttcagcc 29
<210> 16
   <211> 29
   <212> DNA
   <213> Artificial Sequence
<220>
   <223> PCR Primer
<400> 16
   gcggccgcct atttggagaa agaggtcat 29
<210> 17
   <211> 23
   <212> DNA
   <213> Artificial Sequence
<220>
   <223> PCR Primer
<400> 17
   tttttttttc agtgtaaaag gtc 23
<210> 18
   <211> 19
   <212> DNA
   <213> Artificial Sequence
<220>
   <223> PCR Primer
<400> 18
   cagatgacat cctggccag 19
<210> 19
   <211> 22
   <212> DNA
   <213> Artificial Sequence
<220>
   <223> PCR Primer
<400> 19
   ctatacagga aagtatggca gc 22
<210> 20
   <211> 118
   <212> DNA
   <213> Artificial Sequence
<220>
   <223> PCR Primer
<400> 20
   gccaagcttc catgagggcc tggatcttct ttctcctttg cctggccggg agggctctgg      60
   cagcccctca gcaagaagcg ctcgctcaca gccaccgcga cttccagccg gtgctcca       118
<210> 21
   <211> 123
   <212> DNA
   <213> Artificial Sequence
<220>
   <223> PCR Primer
<400> 21
   ccaggtggag caccggctgg aagtcgcggt ggctgtgagc gagcgcttct tgctgagggg     60
   ctgccagagc cctcccggcc aggcaaagga gaaagaagat ccaggccctc atggaagctt    120
   ggc                                                                  123